# EUROPÄISCHE PATENTSCHRIFT

(11) **EP 3 157 427 B1**
(45) Veröffentlichungstag und Bekanntmachung des Hinweises auf die Patenterteilung: **26.08.2026**
(21) Anmeldenummer: 15730968.3
(22) Anmeldetag: 19.06.2015
(51) Int. Cl.: A61B 5/12, H04R 25/00, A61B 5/00

(54) **VERFAHREN UND VORRICHTUNG ZUR UNTERSUCHUNG DES HÖRVERMÖGENS**
METHOD AND DEVICE FOR EXAMINING THE FACULTY OF HEARING
PROCÉDÉ ET DISPOSITIF D'ANALYSE DE LA CAPACITÉ AUDITIVE

(30) Priorität: 20.06.2014 DE 102014108663
(43) Veröffentlichungstag der Anmeldung: 26.04.2017
(73) Patentinhaber: Eberhard Karls Universität Tübingen Medizinische Fakultät, 72074 Tübingen (DE)
(72) Erfinder: ZELLE, Dennis, 72074 Tübingen (DE); DALHOFF, Ernst, 72108 Rottenburg (DE)
(74) Vertreter: Graf von Stosch, Andreas
(86) Internationale Anmeldenummer: PCT/EP2015/001246
(87) Internationale Veröffentlichungsnummer: WO 2015/192969

(56) Entgegenhaltungen:
- EP-A1- 1 027 863
- LUKASHKIN ANDREI N ET AL: "Modifications of a single saturating non-linearity account for post-onset changes in 2&hthinsp;f1-f2 distortion product otoacoustic emission", THE JOURNAL OF THE ACOUSTICAL SOCIETY OF AMERICA, AMERICAN INSTITUTE OF PHYSICS, 2 HUNTINGTON QUADRANGLE, MELVILLE, NY 11747, vol. 112, no. 4, 1 October 2002 (2002-10-01), pages 1561 - 1568, XP012003094, ISSN: 0001-4966, DOI: 10.1121/1.1502903
- ERNST DALHOFF ET AL: "Two-source interference as the major reason for auditory-threshold estimation error based on DPOAE input-output functions in normal-hearing subjects", HEARING RESEARCH, vol. 296, 1 February 2013 (2013-02-01), pages 67 - 82, XP055210511, ISSN: 0378-5955, DOI: 10.1016/j.heares.2012.12.003
- DHAR SUMITRAJIT ET AL: "The effect of stimulus-frequency ratio on distortion product otoacoustic emission components", THE JOURNAL OF THE ACOUSTICAL SOCIETY OF AMERICA, AMERICAN INSTITUTE OF PHYSICS FOR THE ACOUSTICAL SOCIETY OF AMERICA, NEW YORK, NY, US, vol. 117, no. 6, 1 June 2005 (2005-06-01), pages 3766 - 3776, XP012073022, ISSN: 0001-4966, DOI: 10.1121/1.1903846
- WHITEHEAD M L ET AL: "Measurement of otoacoustic emissions for hearing assessment", IEEE ENGINEERING IN MEDICINE AND BIOLOGY MAGAZINE, IEEE SERVICE CENTER, PISACATAWAY, NJ, US, vol. 13, no. 2, 1 April 1994 (1994-04-01), pages 210 - 226, XP011417988, ISSN: 0739-5175, DOI: 10.1109/51.281681
- DALHOFF E ET AL: "Schallund Geschwindigkeits-DPOAE; Technologie, Methodik und Perspektiven", HNO ; DEUTSCHE GESELLSCHAFT FÜR HALS-NASEN-OHREN-HEILKUNDE, KOPFUND HALS-CHIRURGIE, SPRINGER, BERLIN, DE, vol. 58, no. 6, 10 June 2010 (2010-06-10), pages 543 - 555, XP019816198, ISSN: 1433-0458
- DENNIS ZELLE ET AL: "Extraction of otoacoustic distortion product sources using pulse basis functions", THE JOURNAL OF THE ACOUSTICAL SOCIETY OF AMERICA, 1 July 2013 (2013-07-01), United States, pages EL64, XP055210590, Retrieved from the Internet <URL:http://www.ncbi.nlm.nih.gov/pubmed/23862908> DOI: 10.1121/1.4809772]
- SALATA ET AL: "Distortion-product otoacoustic emissions hearing screening in high-risk newborns", OTOLARYNGOLOGY AND HEAD AND NECK SURGERY, ROCHESTER, US, vol. 118, no. 1, 1 January 1998 (1998-01-01), pages 37 - 43, XP005121959, ISSN: 0194-5998, DOI: 10.1016/S0194-5998(98)70372-9

## Beschreibung

Die vorliegende Erfindung betrifft allgemein ein Verfahren zur Untersuchung des Hörvermögens für zumindest ein Ohr eines Säugetiers. Basierend auf der Messung von durch Paare von Anregungssignalen (f1, f2) evozierten DPOAE (Distorsionsprodukte otoakustischer Emissionen) werden für unterschiedliche Anregungsfrequenzen f2 Wachstumskurven ermittelt, mit den Schritten:
dem Ohr werden erste Anregungssignale mit einer ersten Anregungsfrequenz f1 und einem ersten Schallpegel L1 und zweite Anregungssignale mit einer zweiten Anregungsfrequenz f2 und einem zweiten Schallpegel L2 präsentiert; und
in dem Ohr werden Pulspaare mit einem ersten Puls des ersten Anregungssignals und einem zweiten Puls des zweiten Anregungssignals präsentiert und die dadurch evozierten DPOAE erfasst und ausgewertet.
Das erfindungsgemäße Verfahren gemäß Anspruch 1 ist weiterhin dadurch gekennzeichnet, dass ein Block, der zumindest zwei unterschiedliche Pulspaare mit unterschiedlichen zweiten Anregungsfrequenzen f2 aufweist, während einer Messdauer mehrfach wiederholt wird, wobei die Dauer des ersten und des zweiten Pulses in einem Pulspaar 2 bis 20 ms beträgt und wobei jeder Block von Pulspaaren während einer Blockzeit T_{B} präsentiert wird, die so gewählt ist, dass zwischen dem Beginn eines ersten und eines folgenden Pulspaares mit derselben Anregungsfrequenz f2 ein zeitlicher Abstand von 30 bis 100 ms liegt, wobei in einem Block der Beginn eines Pulspaares mit einem zeitlichen Abstand T auf den Beginn des im Block unmittelbar vorhergehenden Pulspaares folgt, wobei T mindestens der Länge des vorausgegangenen Pulses entspricht.

Der Generationsprozess der DPOAE und die bisher verwendeten Mess- und Auswerteverfahren sind beispielsweise beschrieben in Dalhoff et al., "Schall- und Geschwindigkeits-DPOAE", in HNO 2010, 58: 543-555. Dort finden sich auch weitere Nachweise, auf die ausdrücklich verwiesen wird. Luhashkin & Russell (J. Acoust. Soc. Am. 112(4), 2002; pp. 1561 - 1568) beschreiben die Messung von DPOAEs bei Meerschweinchen.

Die bekannten Verfahren dienen zur objektiven und quantitativen Ermittlung der Schallverarbeitung in einem Säugerohr und somit der Untersuchung und nachfolgenden Bewertung des Hörvermögens. Die Verfahren basieren auf der Messung von Distorsionsprodukten otoakustischer Emissionen (DPOAE), die durch Paare von Anregungssignalen erzeugt werden.

Die Messergebnisse können gemäß der EP 2 053 877 A1 und der DE 199 05 743 A1 auch zur Einstellung von Hörgeräten verwendet werden.

Das Hörsystem kann als eine Kette von Verarbeitungsblöcken angesehen werden, die durchlaufen werden, bevor im Cortex die komplexere Wahrnehmung des Hörens entsteht. Die ersten Blöcke sind das äußere Ohr (Ohrmuschel & Gehörgang), das Mittelohr (Gehörknöchelchen mit der Fußplatte als Grenze zu den Flüssigkeiten des Innenohrs), und das flüssigkeitsgefüllte Innenohr. Diese drei Blöcke werden auch die Peripherie genannt, auf sie folgen mehrere neurale Verarbeitungsknoten, bevor die Signale im Cortex ankommen. Zum Innenohr gehört die Hörschnecke (lat. Cochlea), die das Rezeptorfeld für die Hörwahrnehmung darstellt und in der Töne ähnlich einer Fourieranalyse in ihre einzelnen Frequenzen zerlegt werden.

Die meisten Hörschäden entstehen im Innenohr. Darunter fällt auch die Altersschwerhörigkeit, die im Mittel bei 60 bis 70-jährigen Männern zu 35 dB und bei Frauen dieser Altersgruppe zu 25 dB Hörverlust bei Frequenzen ab 4 kHz führt.

Diese Altersschwerhörigkeit ist dominiert durch eine Beeinträchtigung des sogenannten cochleären Verstärkers (die mechanische Verstärkung der Wanderwelle in der Hörschnecke (Cochlea)) im Innenohr, der im gesunden Zustand mit einem komplexen Zusammenspiel von elektro-mechano-biochemischen Mechanismen eine Verstärkung der Vibration im Innenohr um den Faktor 300 bis 1000 erzielt, bevor die Vibrationen von den inneren Haarzellen in neurale Signale umgewandelt werden.

Das Schlüsselelement des cochleären Verstärkers stellen die äußeren Haarzellen dar, die im Prinzip wie Piezoaktoren wirken. Im Gegensatz zu den meisten Schädigungen des Mittelohres können Schäden des cochleären Verstärkers heute noch nicht erfolgreich behandelt werden.

Der Zustand des Mittelohrs kann in der Regel ausreichend mittels Tympanometrie erfasst werden. Der Zustand des gesamten Hörsystems wird subjektiv durch Reintonaudiometrie sowie Sprachverständlichkeitstests und objektiv durch Ableitung neuraler Erregung überprüft.

Bei Ausschluss einer Schädigung der Mittelohrkomponente bleibt bei Vorliegen eines Hörverlusts die Entscheidung zwischen einer neuralen und einer cochleären Störung. Hierzu dient die Messung otoakustischer Emissionen (OAE). OAE sind aktive, akustische Aussendungen des Ohres, die retrograd, d.h. entgegen der Richtung bei der Schallwahrnehmung, über den Weg Gehörknöchelchen und Trommelfell in den Gehörgang gelangen und dort mit Hilfe von hochempfindlichen Messmikrofonen aufgenommen werden können.

Es werden zwei verschiedene Typen von OAE unterschieden, nämlich die spontanen OAE und die durch akustische Reize hervorgerufenen, evozierten OAE. Die spontanen OAE (SOAE) treten bei 35 bis 50 % der gesunden Ohren auf und sind für den Erzeuger selber nicht hörbar und haben keine wesentliche klinische Bedeutung.

Die evozierten OAE (EOAE) entstehen während oder kurz nach einer akustischen Stimulation des Ohres. Je nach Form des akustischen Stimulus werden unterschiedliche Subgruppen der evozierten OAE unterschieden, zu denen insbesondere die transitorisch evozierten OAE (TEOAE), die nach einem kurzen akustischen Stimulus nachweisbar sind, und die Distorsionsprodukt-otoakustischen Emissionen (DPOAE) zählen, die durch zwei simultan applizierte Sinustöne (f1 und f2) erzeugt werden.

Das erfindungsgemäße Verfahren zur Bestimmung des Zustandes der Cochlea basiert auf der Messung der DPOAE, also der Subgruppe der OAE, die besonders intensiv erforscht wurde.

DPOAE sind Nebenprodukte eines gesunden, aktiven Innenohrverstärkers, der beim Menschen und anderen Säugetieren die durch einen akustischen Reiz erzeugten Schwingungen im Innenohr um einen Faktor von 100 bis 1000 verstärkt, bevor die Umsetzung in neurale Signale erfolgt. Der cochleäre Verstärker verwendet hierzu externe Energie, die durch den Metabolismus zur Verfügung gestellt werden muss.

DPOAE werden durch simultane Reizung mit zwei Primärtönen f1 und f2 mit Anregungspegeln L1 und L2 erzeugt. Die zwei Primärtöne f1 und f2 sind dabei sogenannte "pure tones", also Reintöne, die jeweils eben nur eine Frequenz enthalten. Entsprechend der allgemeinen Fourier-Beziehung zwischen Zeit- und Frequenzbereich müssten diese Töne also unendlich lang anhalten, da sich sonst das Spektrum verbreitert. Da dieses unendlich lange Anhalten nicht zu realisieren ist, versteht der Fachmann darunter Töne, die solange präsentiert werden, dass ihr Spektrum scharf ist. Aufgrund einer ausgeprägten Nichtlinearität in der Kennlinie des cochleären Verstärkers kommt es zur Erzeugung von Distorsionsprodukten, die teilweise retrograd zurück in den Gehörgang übertragen werden und dort mit geeigneten Instrumenten gemessen werden können. Dabei wird ein bestimmtes Distorsionsprodukt ausgewertet, welches bevorzugt bei der Frequenz fdp = 2f1 - f2 liegt. Seine Amplitude erlaubt Rückschlüsse über den Zustand des cochleären Verstärkers, die in der allgemeinen klinischen Praxis wertvoll sind, beispielsweise beim Screening Neugeborener auf behandlungsbedürftige Hörschäden.

In der Regel wird die Amplitude des DPOAE mittels Fouriertransformation aus dem Spektrum des gemessenen Signals extrahiert. Da die DPOAE einen sehr geringen Schallpegel aufweisen, der deutlich unter der Hörschwelle liegen kann, muss ausreichend lange gemittelt werden, um einen gewissen Rauschabstand und damit eine zuverlässige diagnostische Aussage zu erhalten.

In diagnostischen Anwendungen wird das Frequenzverhältnis f2/f1 bevorzugt konstant gehalten, und zwar deswegen, weil es in jeder Spezies ein Frequenzverhältnis gibt, bei dem das DPOAE am stärksten und damit leichtesten messbar ist. Beim Menschen ist dieses Verhältnis 1,2 und entspricht damit einer kleinen Terz.

Der Hauptteil des DPOAE-Signals wird dort im Innenohr produziert, wo eigentlich f2 abgebildet wird. Dies liegt daran, dass dort die Wanderwelle von f2 maximal ist, und die Wanderwelle vom f1-Ton an diesem Ort auch schon recht stark ist. Am weiter apikal liegenden Ort des Maximums der f1-Wanderwelle ist dagegen die f2-Welle bereits vollkommen zusammengebrochen. Daher ist der f2-Abbildungsort derjenige, an welchem beide Töne relativ stark sind und also gleichzeitig von den stark nichtlinearen Kennlinien der Ionenkanäle der äußeren Haarzellen prozessiert werden.

Der Fachmann und auch der HNO-Arzt assoziiert ein DPOAE-Ergebnis daher immer mit der f2-Frequenz, vergleicht also ein mit f2 = 3 kHz stimuliertes DPOAE mit dem Audiogramm bei 3 kHz, obwohl die Frequenz des DPOAE selber ziemlich genau zwei Terzen darunter liegt.

Wenn man mehrere solche DPOAE bei einer Frequenz f2 und verschiedenen Schallpegeln L2 misst, und zu einer sogenannten Wachstumsfunktion zusammenfügt, ergibt sich eine genauere Aussage über die Funktion des cochleären Verstärkers im Innenohr. Für jede Frequenz f2 kann dann anhand der Wachstumskurve der sogenannte Schwellwert bestimmt werden, unter dem der geringste Anregungspegel L2 verstanden wird, bei dem das DPOAE noch einen gegebenen minimalen Rauschabstand erreicht. Diese Schwelle kann nicht gemessen werden, da das mitgemessene Rauschen endlich ist, sondern muss durch Extrapolation bestimmt werden.

Um eine diagnostische Aussage über den gesamten Frequenzbereich zu erhalten, werden daher typischerweise 6 bis 8 Wachstumsfunktionen sequentiell gemessen.

Die so bestimmten Wachstumskurven und daraus extrapolierten Schwellwerte können dann als Basis für eine verbesserte Diagnostik und zur Einstellung von Hörhilfen dienen, weil die extrapolierten Schwellwerte als unmittelbare Aussage über den Hörverlust anzusehen sind, siehe die eingangs erwähnte DE 199 05 743 A1.

Die Anregung der DPOAE kann dabei sowohl durch kontinuierliche Töne als durch gepulste Töne erfolgen. Wie bereits vorhergehend erwähnt, versteht man dabei unter kontinuierlichen Tönen Töne, die solange präsentiert werden, dass ihr Spektrum scharf ist. Bei der gepulsten DPOAE wird f1 als Dauerton oder gepulst und f2 gepulst eingespeist, wobei das Verhältnis von L2 zu L1 in einem bestimmten Bereich eingestellt und dann L2 schrittweise verändert wird. Bei den gepulsten Tönen handelt es sich nach der allgemeinen Fourier-Beziehung zwischen Zeit- und Frequenzbereich um Töne, deren Spektrum aufgrund der Kürze des Pulses verbreitert ist. Wird einer der Töne, wie z.B. vorerwähnter Ton f1, als Dauerton präsentiert, bedeutet das, dass der gepulst eingespeiste Ton f2 während des Anhaltens des Tones f1 einen An- und Ausschaltprozess durchläuft.

Durch die Messung von Wachstumsfunktionen mittels gepulster DPOAE können gewisse Artefakte, die bei Dauerton DPOAE als "Zweiquellenproblematik" bekannt sind, unterbunden werden.

Das Verfahren beruht auf zwei Schlüsselkomponenten: 1) Extraktion der sog. nichtlinearen Komponente der DPOAE mittels gepulster Stimulation und zeitlicher Isolation; 2) Messung von Wachstumsfunktionen von gepulsten DPOAE mit Anwendung des Extrapolationsverfahren von Boege & Janssen, mit der wichtigen Modifikation der "high-level saturation correction". Beide Verfahren sind beschrieben in Dalhoff et al., "Two-source interference as the major reason for auditory-threshold estimation error based on DPOAE input-output functions in normal-hearing subjects", in Hearing Research 296 (2013), Seiten 67 - 82.

Mit der Verbesserung dieses Verfahrens beschäftigt sich die vorliegende Erfindung im Einzelnen.

Das bekannte Verfahren zeigt nämlich einen Nachteil, der die Anwendung in der HNO-Routine verhindert. Die Schätzung der Hörschwelle bei nur einer Frequenz dauert bei den bekannten Verfahren etwa 480 s; wobei naheliegende Maßnahmen zur Reduzierung dieser Messzeit auf 96 s diskutiert wurden. Soll der übliche Satz von sieben Frequenzen zur klinischen Beschreibung des Hörvermögens getestet werden, so ergibt sich schon für Normalhörende eine Messzeit von 11 min.

Ein wesentlicher Nachteil des bekannten Verfahrens besteht also darin, dass es sehr langsam ist, und daher bisher als nicht klinisch anwendbar galt, insbesondere weil es für die Untersuchung von Hörgeschädigten viel zu langwierig ist. Zudem ist die erzielbare Genauigkeit bei der Bestimmung der Wachstumskurve und der extrapolierten Schwellwerte häufig nicht zufriedenstellend.

Hier setzt das erfindungsgemäß weiterentwickelte Verfahren der schnellen Puls-Distorsionsprodukt-otoakustischen Emissionen (Puls-DPOAE) an, das sich die Aufgabe stellt, ein schnell durchzuführendes und dennoch genaues Verfahren der eingangs genannten Art zu schaffen.

Erfindungsgemäß wird diese Aufgabe bei dem eingangs genannten Verfahren dadurch gelöst, dass ein Satz von zumindest zwei unterschiedlichen Pulspaaren (jedes Pulspaar mit einer Anregungsfrequenz f1 und f2) mit unterschiedlichen zweiten Anregungsfrequenzen f2 (und folglich auch unterschiedlichen ersten Anregungsfrequenzen f1) in einem Block präsentiert wird, der während einer Messdauer mehrfach wiederholt wird. Ein Beispiel für zwei unterschiedliche Pulspaare eines Blocks sind ein erstes Pulspaar mit einer Anregungsfrequenz f2 von 1,5 kHz und einer Anregungsfrequenz f1 von 1,25 kHz und ein zweites Pulspaare mit einer Anregungsfrequenz f2 von 4 kHz und einer Anregungsfrequenz f1 von 3,33 kHz. Die Anregungsfrequenzen f2 und f1 eines Pulspaares sind dabei vorzugsweise über ein Frequenzverhältnis f2/f1 = 1,2 verknüpft. Abweichend von diesem Frequenzverhältnis f2/f1 von 1,2 kann dieses Frequenzverhältnis jedoch auch auf einen anderen geeigneten Wert zwischen 1,15 und 1,35 festgelegt werden. Da vorliegend jedoch eine Konzentration auf die Schwellwertbestimmung erfolgt, ist ein Frequenzverhältnis f2/f1 von 1,2 oder 1,22 günstig, da nach gegenwärtigem Kenntnisstand das Frequenzverhältnis bei niedrigen Anregungsschalldrücken kaum frequenzabhängig ist. Wenn nachfolgend von f2-Pulspaaren gesprochen wird, sind dabei immer f1-f2 Pulspaare gemeint, bei denen die Frequenz f1 über ein definiertes Frequenzverhältnis aus f2 bestimmt wird; und wenn in diesem Zusammenhang von dem Anregungspegel L2 gesprochen wird, wird davon ausgegangen, daß der Pegel L1 des f1-Pulses nach einer vordefinierten Regel aus L2 berechnet wird.

Gepulste DPOAE haben gegenüber kontinuierlichen DPOAE zunächst den gravierenden Nachteil, dass die Messung bei einer Frequenz-Schallpegelkombination generell eine geringe Zeitauslastung und damit ein entsprechend geringeren Rauschabstand bei derselben Messzeit aufweist, weshalb die Verwendung von Pulspaaren auf den ersten Blick die Messzeit sogar zu verlängern scheint.

Dieser Nachteil wird jedoch erfindungsgemäß erheblich reduziert, indem mehrere Messungen im Zeit-Frequenzraum miteinander verschränkt werden, d.h. zeitversetzt abwechselnd präsentiert werden. So können innerhalb eines Blocks zeitversetzt bspw. sieben Frequenzen stimuliert und analysiert werden.

Die der Erfindung zugrunde liegende Aufgabe wird durch das beanspruchte Verfahren vollkommen gelöst.

Erfindungsgemäß wird eine Beschleunigung des Verfahrens durch parallele und adaptive Stimulations- und Analyseschritte erreicht. Mit dem neuen Verfahren wurden in aktuellen Versuchen der Erfinder Wachstumskurven für 5 Frequenzen f2 in typischerweise 2,5 min gemessen. Schätzungen zeigen, dass mit dem Verfahren wachstumskurven für alle sieben Frequenzen in 2 min gemessen werden können.

Die Erfinder haben zudem ein Schwellenannäherungsverfahren konzipiert, das darauf abzielt, adaptiv während einer individuellen Messung so wenig Punkte der Wachstumsfunktion wie möglich zu messen, vorzugsweise 3 oder 4, um Zeit zu sparen ohne Genauigkeit zu verlieren.

Dazu müssen die Anregungspegel L2 je nach Patient und frequenzabhängigen Zustand seines Gehörs gewählt werden.

Die Erfinder haben zudem mit dem neuen Verfahren den aus ihrer Sicht bislang besten Weg aufgezeigt, wie durch physiologische Prozesse bedingte Artefakte (erzeugt z.B. durch Herzschlag, Atmung, Schlucken, Husten, Reflexe des Muskels im Mittelohr) in einem dennoch schnellen Messverfahren zuverlässig unterdrückt werden können.

Die Erfinder konnten damit erstmals zeigen, dass mit dem neuen Verfahren eine Schwellenschätzung des cochleären Zustands mit einer Standardabweichung von nur 3 bis 4 dB möglich ist, wobei einzelne Schwellenschätzungen nicht mehr als 10 dB Fehler aufweisen.

Während die typischerweise sechs bis acht Wachstumsfunktionen bisher sequentiell gemessen wurden, erfolgen diese Messungen jetzt erfindungsgemäß verschränkt.

Im Prinzip wird mit dem Pulsverschränkungsverfahren der Nachteil behoben, dass gepulste Signale nicht die gesamte Zeit eines Messblocks nutzen und damit SNR verlieren.

Hierzu wird während der laufenden Messung der Rauschabstand des Spektrums auf der Basis der bereits registrierten Signalblöcke ermittelt. Sobald der gewünschte Rauschabstand erreicht ist, wird die Messung abgebrochen. Da das Hintergrundrauschen aufgrund physiologischer Prozesse, wie etwa Herzschlag, Atmung, Schlucken, zeitlich stark variiert, wird eine Rauschbewertung jedes einzelnen Blocks während der Messung durchgeführt, und einzelne Blöcke werden verworfen, wenn das Rauschen einen gewissen, empirisch ermittelten Schwellwert übersteigt.

Die Erfindung betrifft ferner gemäß Anspruch 12 eine Vorrichtung zur Durchführung des neuen Verfahrens, die zumindest eine im/am Ohr zu platzierende Ohrsonde mit einem oder zwei Miniaturlautsprechern und einem Empfänger (Mikrophon) umfasst, wobei jeder Miniaturlautsprecher zur Präsentation eines ersten Anregungssignales mit einer ersten Anregungsfrequenz f1 und einem ersten Schallpegel L1 und/oder eines zweiten Anregungssignales mit einer zweiten Anregungsfrequenz f2 und einem zweiten Schallpegel L2 ausgelegt ist, und wobei der Empfänger zur Erfassung und Weiterleitung eines durch das erste und zweite Anregungssignal evozierten DPOAE ausgelegt ist, wobei ferner eine Rechnereinheit vorgesehen ist, die dazu programmiert und eingerichtet ist, einen Satz von zumindest zwei Pulspaaren mit unterschiedlichen zweiten Anregungsfrequenzen f2 in einem Block zu präsentieren, der für eine Messdauer mehrfach wiederholt durchlaufen wird, so dass die Pulspaare eines Satzes verschränkt, d.h. zeitversetzt abwechselnd präsentiert werden.

Der erste und der zweite Puls in einem Pulspaar werden erfindungsgemäß gleichzeitig oder geringfügig zeitversetzt präsentiert. Normalerweise wird der f1-Puls frequenzabhängig 3-10 ms früher eingeschaltet, und 3-10 ms später ausgeschaltet, so dass die f1-Anregung während der Präsentation des f2-Pulses kurzzeitig einen Gleichgewichtszustand erreicht. Größere Zeitversätze bedeuten dabei immer einen möglichen Zeitverlust. Es kann aber auch zur größtmöglichen Zeitersparnis mit zwei gleich oder ähnlich kurzen Pulsen für f1 und f2 gearbeitet werden, die so zeitversetzt werden, dass am diagnostisch wertvollsten Abbildungsort für f2 in der Cochlea beide Anregungen gleichzeitig stattfinden. Dann wird der f1-Puls etwa 0.1-3 ms später angeschaltet, da seine Laufzeit zum näher basal (Richtung Fußplatte) gelegenen Abbildungsort der Frequenz f2 kürzer ist als für die f2-Welle. Ist diese Einstellung optimal gewählt, tritt noch kein Effekt durch die afferent-efferente Rückkopplungsschleife des medialen olivocochleären Reflexes ein.

Die Anregungsfrequenz f1 bestimmt sich aus einer vorgegebenen Anregungsfrequenz f2 nach einem vorgegebenen Verhältnis zwischen f1 und f2, wie bereits in Paragraph [0030] beschrieben.

Das Verhältnis beträgt, wie bereits in Paragraph [0030] beschrieben, vorzugsweise f2/f1 = 1,2, weil bei diesem Verhältnis erfahrungsgemäß eine möglichst starke DPOAE erhalten wird. Das Verhältnis kann zur weiteren Optimierung je nach f2 und L2 leicht angepasst werden, siehe z.B. Johnson et al. "Influence of primary-level and primaryfrequency ratios on human distortion product otoacoustic emissions", in J. Acoust. Soc. Am. 119, 2006, Seiten 418 - 428;

Die ersten und zweiten Anregungsfrequenzen liegen vorzugsweise im Bereich von 250 Hz bis 10 kHZ.

Die Dauer des ersten und des zweiten Pulses in einem Pulspaar beträgt erfindungsgemäß 2 bis 20 ms. Der Puls mit der Anregungsfrequenz f1 eines Pulspaares setzt dabei ein bevor der Puls mit der Anregungsfrequenz f2 einsetzt, und endet nachdem der Puls mit der Anregungsfrequenz f2 beendet wurde, d.h. der Puls mit der Anregungsfrequenz f1 ist länger als der Puls der Anregungsfrequenz f2 eines Pulspaares. Es versteht sich, dass diese Verhältnisse auch genau umgedreht werden können, nämlich dass der Puls mit der Anregungsfrequenz f2 einsetzt, bevor der Puls mit der Anregungsfrequenz f1 einsetzt und dass der Puls mit der Anregungsfrequenz f1 endet bevor der Puls mit der Anregungsfrequenz f2 endet, so dass der Puls der Anregungsfrequenz f2 länger ist als der Puls der Anregungsfrequenz f1 eines Pulspaares. Unter Pulslänge verstehen die Erfinder dabei die sogenannte volle Halbwertsbreite (T_{HB} bzw. "full width half maximum" (T_{FWHM}). Für diese Pulslänge wird die Zeit bestimmt, ab der die kosinusförmige Anstiegsflanke auf die Hälfte des Gleichgewichtswerts ("steady-state") angestiegen ist, bis zu dem entsprechenden Zeitpunkt in der Abschaltflanke.

Diese Pulslänge ergeben sich aus der bevorzugten Pulsform, gemäß der die Pulse einen kosinusförmigen Anstieg von 0,1 bis 4 ms Länge, einen steady-state mit dem Pegel L2 bzw. L1, der 2 bis 12 ms lang ist, gefolgt von einem weiteren kosinusförmigen Abschnitt.

Die Gesamtlänge eines Pulses wird in einer bevorzugten Anordnung so bemessen, dass er seine volle Amplitude erreicht, während die Antwort des ersten Beitrags zum DPOAE, d.h. des nichtlinearen Beitrags, beginnt, aber bereits im Abklingprozess begriffen ist, wenn die Antwort des zweiten Beitrags zum DPOAE beginnt.

Auf diese Weise werden zwei Ziele erreicht, nämlich möglichst wenig Zeit für die Messung des ersten (nichtlinearen) Beitrags eines DPOAE zu verwenden, und ihn sauber von dem zweiten Beitrag zu trennen. Mögliche Pulsformen sind in Whitehead et al., "Visualization of the onset of distortion-product otoacoustic emissions, and measurement of their latency", in J. Acoust. Soc. Am. 100 (3), 1996, Seiten 1663 - 1679, und Zelle et al., "Extraction of otoacoustic distortion product sources using pulse basis functions", in Acoust. Soc. Am. 134, 2013, Seiten EL64-69, beschrieben.

Zur effektiven Unterdrückung der Primärtöne (f1 und f2) wird neben üblichen Filtermethoden vorzugsweise das Primary-tone-phase-variation-Verfahren von Whitehead et al., 1996, a.a.O., eingesetzt.

In einem Block folgt der Beginn eines Pulspaares mit einem zeitlichen Abstand T (T_{SLOT} oder T_{S}) auf den Beginn des im Block unmittelbar vorhergehenden Pulspaares, wobei T in der Regel mindestens der Länge des vorausgegangenen Pulses entspricht, also > 10 ms ist. Diese für ein Pulspaar in einem Block reservierte Messzeit wird nachfolgend auch als Slot bezeichnet. Es ist dabei zu bemerken, dass Slots sich nicht überlappen, sondern ein Slot dem anderen folgt, wenn der vorhergehende Slot beendet ist. Wie vorhergehend ausgeführt, beträgt die Slotlänge (T_{S}) dabei mehr als 10 ms.

Hier ist von Vorteil, dass auf diese Weise das zweite Pulspaar erst präsentiert wird, wenn die durch das erste Pulspaar evozierte DPOAE hinreichen (auf ca. 1 bis 10% des Ausgangswertes) abgeklungen ist, so dass es zu keinen merklichen Störeinflüssen bei der Messung der Pegel (Ldp) der einzelnen DPOAE kommt, die verglichen mit den Schallpegeln L1 und L2 der Anregungspulse nur einen sehr geringen Schalldruckpegel (SPL) aufweisen. Zudem ermöglicht der erhöhte zeitliche Abstand zwischen der Präsentation von Pulspaaren mit gleichen Anregungsfrequenzen f1 und f2 eine hinreichende Erholungszeit zum vollständigen Abklingen des im vorhergehenden Messblock ausgelösten DPOAE.

Wird beispielsweise bei einer Messung mit vier Pulspaaren für jedes Pulspaar in einem Block ein Abstand T bzw. eine Slotlänge TS = 40 ms gewählt, dann ergibt sich für jede DPOAE einer Anregungsfrequenz f2 eine Erholungszeit von 3 x 40 ms, also 120 ms, bevor auf derselben Anregungsfrequenz f2 die Messung wiederholt wird. Innerhalb einer sich so ergebenden gesamten Blockzeit (T_{B}) von 160 ms (4 x 40 ms = 4 x T_{S}) können in diesem Beispiel so DPOAE für vier zweite Anregungsfrequenzen f2 gemessen werden, ohne dass diese sich gegenseitig oder durch zu geringe Abklingzeit beeinflussen. Der Block weist in diesem Beispiel also vier Slots mit einer Slotlänge T_{S} von je 40 ms auf, wobei die aktuelle Belegung der Slots mit Pulspaaren unterschiedlicher zweiter Anregungsfrequenzen f2 als Panel bezeichnet wird.

Die Gefahr der gegenseitigen Beeinflussung der Pulspaare sowie der von ihnen evozierten DPOAE in einem Block besteht, weil bei gleichzeitiger Präsentation von Stimuli die Wanderwellen die nichtlineare Übertragung im Innenohr in Saturation treiben können, was als Suppression oder auch Maskierung bezeichnet wird. Die Messung insbesondere von gleichzeitig präsentierten höheren Frequenzen wird dadurch verfälscht.

Weiter ist es bevorzugt, wenn in einem Block die zweiten Anregungsfrequenzen f2 von zwei unmittelbar aufeinander folgenden Pulspaaren, also in zwei aufeinanderfolgenden Slots, mindestens eine Oktave auseinanderliegen. Die ersten Anregungsfrequenzen f1 werden dabei, wie in Paragraph [0030] beschrieben, gewählt.

Auch hier ist von Vorteil, dass es zu keinen merklichen Störeinflüssen bei der Messung der einzelnen DPOAE kommt, weil dafür gesorgt wird, dass der Frequenzabstand zwischen den Anregungsfrequenzen f1, f2 und damit auch den Frequenzen fdp der jeweils evozierten DPOAE hinreichend groß ist. Es hat sich gezeigt, dass ein Frequenzabstand von einer Oktave ausreichend ist.

Ein bevorzugter Satz (bevorzugtes Panel) besteht aus den Anregungsfrequenzen f2 = 1 kHz, f2 = 3 kHz, f2 = 1,5 kHz, f2 = 6 kHz, die als Panel in einem Block in dieser Reihenfolge wiederholt präsentiert werden. Ein Panel ist zu verstehen als das Frequenzzeitmuster der Anregungsfrequenzen in einem Block.

Ein anderer bevorzugter Satz (bevorzugtes Panel) besteht aus den Anregungsfrequenzen f2 = 2 kHz, f2 = 4 kHz, f2 = 1,5 kHz, f2 = 3 kHz, die als Panel in einem Block in dieser Reihenfolge wiederholt präsentiert werden. Weil für die Messung bei 1 kHz typischer Weise doppelt soviel Zeit benötigt wird, um einen gewissen Rauschabstand zu erreichen, wie für die Messung bei den anderen (höheren) Frequenzen, wird typischer Weise für diesen Satz eine kürzere Messdauer bis zum Erreichen eines gewünschten Signal-Rausch-Verhältnisses (SNR) benötigt.

Allgemein ist es bevorzugt, wenn während der Messdauer die gemessenen Schallpegel Ldp der DPOAE für Pulspaare gleicher erster und zweiter Anregungsfrequenzen f1, f2 gemittelt werden.

Hier ist von Vorteil, dass die Messungen mit den Blöcken des Satzes von Pulspaaren über einen gewissen Zeitraum, der Messdauer wiederholt werden, weil das SNR verbessert wird, wenn die DPOAE für Pulspaare gleicher Anregungsfrequenzen f1, f2 gemittelt werden.

Der oder jeder Block von Pulspaaren wird während einer Blockzeit T_{B} präsentiert, die so gewählt ist, dass zwischen dem Beginn eines ersten und eines folgenden Pulspaares mit derselben Anregungsfrequenz f2 ein zeitlicher Abstand von 30 bis 100 ms, vorzugsweise von zumindest 70 ms liegt. Wie bereits erläutert ist die Blockzeit T_{B} dabei die Summe der Slotlängen T_{S} eines Blocks.

Nach den Erkenntnissen der Erfinder ist dieser zeitliche Abstand ausreichend, damit ein Puls ausreichend angeklungen ist, wenn er wiederholt wird

Allgemein ist es bevorzugt, wenn zu Beginn der Messungen überprüft wird, ob die Frequenz fdp einer der DPOAE mit einer spontanen Emission (SOAE) interferiert.

Hier ist von Vorteil, dass Artefakte und Störquellen schon zu Beginn der Messung erkannt werden. Ist dies der Fall, kann entweder die Blockzeit T_{B} bzw. die Zeitdauer T_{S} für einen oder alle Slots so angepasst werden, dass die Abklingzeit des DPOAE so weit verlängert wird, dass sein Pegel unter eine gewisse Schwelle gelangt, bevor das nächste Pulspaar präsentiert wird, oder die Frequenz f2 wird verschoben, um einen Mindestabstand zu der SOAE herzustellen.

Dabei ist es bevorzugt, wenn zu Beginn einer Messung für ein Pulspaar mit einer ersten Anregungsfrequenz f1 und einem ersten Schallpegel L1 und einer zweiten Anregungsfrequenz f2 und einem zweiten Schallpegel L2 ein DPOAE gemessen wird, und für den Fall, dass kein DPOAE messbar ist, der Schallpegel L2 (und der Schallpegel L1) inkremental erhöht wird, bis entweder der maximal ausgebbare Schallpegel L2 (L1) erreicht ist oder ein DPOAE gemessen wird. Auf diese einfache Weise kann die Anwesenheit störender SOAE erkannt und ggf. kompensiert werden.

Weiter ist es bevorzugt, wenn innerhalb eines Blocks die Schallpegel der Pulspaare ähnlich gewählt werden, da mit zunehmender Pegeldifferenz die Gefahr der gegenseitigen Suppression zunimmt.

In einer einfachen Ausführung kann für die maximale Pegeldifferenz zwischen den L2-Schallpegeln von beispielsweise vier Pulspaaren in einem Block eine feste Schwelle definiert werden, die in einer bevorzugten Ausführung zwischen 5 und 15 dB liegt.

Einerseits ist es bevorzugt, wenn die Abfolge der Pulspaare und der zeitliche Abstand zwischen zwei aufeinanderfolgenden Pulspaaren, also die Slotzeit (T_{S}), in einem Block konstant sind.

Bei dieser blockstarren Vorgehensweise werden so viele Blöcke gemessen und gemittelt, bis für jede Anregungsfrequenz in dem Satz (Panel) das gewünschte SNR erreicht ist.

Andererseits ist es bevorzugt, wenn bei Erreichen des gewünschten SNR für eine Anregungsfrequenz f2 die noch vorgesehenen Pulspaare für diese Anregungsfrequenz f2 und folglich deren Mittelungen übersprungen werden.

Hier ist von Vorteil, dass mit den noch verbleibenden Pulspaaren in verkürzten Blöcken, also mit weniger Slots, weiter gemessen wird, was die Messzeit weiter verkürzt.

Weiter ist es bevorzugt, wenn zumindest zwei Sätze mit zumindest teilweise hinsichtlich der zweiten Anregungsfrequenz f2 unterschiedlichen Pulspaaren ausgewählt werden, wobei die Blöcke der einzelnen Sätze zeitlich nacheinander präsentiert und die DPOAE gemessen und gemittelt werden. Die Sätze werden also nacheinander abgearbeitet.

In diesem blockflexiblen Verfahren mit fester Pulsanordnung werden beispielsweise sieben Pulspaare mit unterschiedlichen Anregungsfrequenzen f2 so angeordnet, das sie nach allgemeiner Erfahrung alle etwa die Mittelungszeit zugeteilt bekommen, die benötigt wird, um einen bestimmten Rauschabstand zu erzielen.

So enthält der erste Satz etwa vier Pulspaare obwohl insgesamt sieben gemessen werden sollen. Würden alle sieben Pulspaare zu einem Block zusammengefasst, so wäre die Erholungszeit für jede einzelne Frequenz übertrieben groß, z.B. 6 x 40 ms = 240 ms.

Um einen sinnvollen Kompromiss zwischen kürzester Messzeit und ausreichend Zeit zum Abklingen jedes DPOAE zu erhalten, werden erfindungsgemäß mehrere Sätze nacheinander präsentiert, auf die die beispielsweise sieben Pulspaare so verteilt werden, dass Pulspaare mit niedrigen Anregungsfrequenzen f2 (und damit auch niedrigen Anregungsfrequenzen f1) in mehreren Sätzen vorkommen.

Weil bei zu niedrigen Anregungsfrequenzen f2 das Hintergrundrauschen (1/f-Rauschen) ansteigt, führt diese Zuordnung dazu, dass DPOAE für Pulspaare mit tieferen Anregungsfrequenzen f2 häufiger gemittelt werden als solche für höhere Anregungsfrequenzen f2. Dies führt noch einmal zu einer verringerten Messzeit.

Sofern bei einem Patienten jedoch einer der Anregungsfrequenzen f2 nur zu einer sehr schwachen DPOAE beispielsweise bei f2 = 3 kHz führt, muss trotz des guten Rauschhintergrundes bei dieser Anregungsfrequenz viel länger gemittelt werden als üblich, weshalb auch die anderen Anregungsfrequenzen f2, die in dem gemessenen Satz ebenfalls präsentiert werden, unnötig lange stimuliert werden, was die Messzeit in derartigen Situationen wieder verlängern könnte.

In einer Weiterbildung ist es daher bevorzugt, wenn für jedes Pulspaar laufend überprüft wird, ob ein gewünschtes SNR erreicht wird, und wenn bei der weiteren Messung die Pulspaare für diese Anregungsfrequenz f2 eliminiert und die verbleibenden Pulspaare ggf. neu auf die Blöcke verteilt werden.

Bei diesem blockflexiblen Verfahren mit freier Pulspaaranordnung wird die Länge der Messungen für die einzelnen Pulspaare nicht mehr relativ zueinander festschrieben. Zwar wurde auch bei einigen der bisher beschriebenen Verfahrensvarianten nur solange gemittelt, bis für jede Anregungsfrequenz f2 das gewünschte SNR erreicht war, aber das bedeutete, das alle Pulspaare auf das letzte Pulspaar warten mussten, so dass alle Anregungsfrequenzen f2 bis auf eine ein höheres SNR aufwiesen als gefordert, was durch eine zu hohe Messzeit erkauft wird.

Erfindungsgemäß wird daher jetzt für jedes Pulspaar laufend überprüft, ob das SNR erreicht ist; sobald das der Fall ist, wird geprüft, ob noch ein weiteres Pulspaar unvollendet ist. Auf diese Weise werden die fertig gemessenen Pulspaare nacheinander aus der Messung eliminiert, und nur die verbleibenden Pulspaare weiter präsentiert.

Wobei jetzt zum einen überprüft wird, ob der Oktavabstand zwischen zwei aufeinander folgenden Pulspaaren eingehalten wird. Ist dies nicht mehr der Fall, werden Pulspaare u.U. nicht mehr in den Blöcken abgearbeitet, denen sie ursprünglich zugeordnet wurden, sondern in anderen (neu definierten) Blöcken. Zudem wird überprüft, ob der erforderliche zeitliche Abstand T (entsprechend der Zeit für einen Slot (T_{S}) zzgl. der notwendigen Abklingzeit (T_{Abkl.})) zwischen Pulspaaren mit gleicher Anregungsfrequenz f2 eingehalten wird.

Vorzugsweise wird die DPOAE für alle in dem oder den Sätzen enthaltenen Anregungsfrequenzen f2 bei einem jeweils der Anregungsfrequenz zugeordneten Schallpegel L2 gemessen und gemittelt, und dann zumindest eine erneute Messung bei neuen Schallpegeln L2 durchgeführt, wobei bevorzugt in einem Schwellwertannäherungsverfahren aus den gemessenen DPOAE für jede Anregungsfrequenz f2 der neue Schallpegel L2 für die jeweils erneute Messung bestimmt wird.

Dieses Verfahren wird wiederholt, bis für jede Anregungsfrequenz f2 eine Wachstumskurve aus Messwerten der Schallpegel der DPOAE für 3 bis 4 unterschiedlichen Schallpegeln L2 bestimmt werden kann, aus der dann die jeweiligen Schwellwerte bestimmt werden.

Typischerweise wird basierend auf dem ersten gemessenen DPOAE für jede Anregungsfrequenz f2 berechnet, was der nächst niedrigere Schallpegel L2 sein soll. Wenn mit einem L2 von 45-55 dB SPL gestartet wurde, ist es höchst wahrscheinlich, dass der folgende nächstniedrigere Schallpegel zwischen 35 bis 50 dB SPL liegt. Halten die sieben berechneten neuen Schallpegel die vorgegebene Bedingung für den maximalen Schallpegelabstand zwischen zwei Pulspaaren ein, kann das vorher verwendete Verfahren wieder angewendet werden, um für jede Wachstumskurve an der Stelle des neuen Schallpegels L2 einen Mittelwert des Schallpegel Ldp der DPOAE zu bestimmen.

in diesem Zusammenhang ist es bevorzugt, wenn die Wachstumskurve aus zumindest drei bei drei unterschiedlichen Schallpegeln L2 aber derselben An-regungsfrequenz f2 bestimmten Werten für Schallpegel Ldp der DPOAE bestimmt wird, wobei die zumindest drei Schallpegel L2 einen oberen Schallpegel L2 umfassen, mit dessen Hilfe ein unterer Schallpegel L2 bestimmt wird, wobei der dritte, mittlere Schallpegel mit Hilfe des oberen und des unteren Schallpegels festgelegt wird, und wenn vorzugsweise mit Hilfe der Formeln (1) bis (14) aus dem nachfolgenden Abschnitt 2.2.3 aus dem oberen Schallpegel L2 und Populationsdaten ein vorläufiger unterer Schallpegel L2 bestimmt wird, und danach aus dem oberen Schallpegel L2 und dem vorläufigen unteren Schallpegel der mittlere Schallpegel L2 bestimmt wird, der vorzugsweise mittig zwischen dem oberen und dem vorläufigen unteren Schallpegel L2 liegt, und wenn weiter vorzugsweise mit Hilfe der Formeln (1) bis (14) aus dem oberen Schallpegel L2 und dem mittleren Schallpegel L2 ein endgültiger unterer Schallpegel L2 bestimmt wird.

Dieses iterative Verfahren wählt erst den oberen Schallpegel L2, schätzt dann mit Hilfe von Populationsdaten und dem oberen Schallpegel einen unteren Schallpegel, bestimmt dann den mittleren Schallpegel zwischen dem oberen und dem vorläufigen unteren Schallpegel, und bestimmt danach aus dem oberen und dem mittleren Schallpegel erneut den unteren, schwellnächsten Schallpegel mit Hilfe der individuellen Steigung, die nun bestimmt werden kann.

Es ist jedoch möglich, dass für eine oder mehrere Anregungsfrequenzen f2 die Schallpegelbedingung nicht erfüllt ist. Es ist prinzipiell auch möglich, dass das Schwellenannäherungsverfahren zu dem Ergebnis kommt, das für einige Anregungsfrequenzen f2 ein niedrigerer Schallpegel L2 festgesetzt werden soll, für andere Anregungsfrequenzen f2 keine DPOAE gemessen werden konnte und daher jetzt mit einem höheren Schallpegel L2 angeregt werden muss.

Die Blöcke müssen dann entsprechend angepasst werden, indem neue Panel festgelegt werden, wobei es vorkommen kann, dass für bestimmte Anregungsfrequenzen f2 Einzelmessungen durchgeführt werden müssen.

Wenn ein Patient beispielsweise einen Hörverlust nur bei 6 kHz zeigt, so kann nach der ersten Präsentation bei einem Startpegel von bspw. 45 dB SPL nichts gemessen werden.

Die Anregungsfrequenz f2 = 6 kHz wird bei einem blockflexiblen Verfahren mit fester Pulsanordnung dann nicht präsentiert. Sind alle Schallpegel L2 der restlichen Anregungsfrequenzen f2 abgearbeitet, folgen bis zu drei Einzelmessungen mit einer Blocklänge von 70 bis 120 ms, um auch bei f2 = 6 kHz zu einer Schwellwertbestimmung zu gelangen.

Bei einem blockflexiblen Verfahren mit freier Pulsanordnung kann dagegen flexibler operiert werden. Hier werden ausstehende Pulspaare permanent auf ihre Kompatibilität hinsichtlich Schallpegel- und Zeitabstand überprüft. Es wird ferner überprüft, ob ausstehende Pulspaare in freigewordene Slot(s) verlagert werden können. Dadurch kann in vielen Fällen die Zahl der Einzelmessungen reduziert werden.

Das neue Verfahren und die neue Vorrichtung können kommerziell eingesetzt werden beim Neugeborenen-Hörscreening, von Kinderärzten bei den Ux-Untersuchungen, in HNO-Kliniken, und von HNO-Ärzten, und zwar jeweils zur Untersuchung des Hörvermögens.

Sie können auch verwendet werden von Hörgeräteakustikern für die Anpassung von Hörgeräten, die jetzt nicht mehr iterativ sondern automatisiert erfolgen kann, und von Patienten, die zuhause einen automatisierten Hörtest durchführen können, um beispielsweise zu überprüfen, ob sie einen Arzt oder eine Klink aufsuchen müssen, oder ob ihr Hörgerät neu eingestellt werden muss.

Es ist auch angedacht, die neue Vorrichtung in Hörgeräte zu integrieren, wo sie *in situ* zur automatischen Anpassung des damit ausgerüsteten Hörgerätes an eine veränderte Hörfähigkeit des Trägers des Hörgerätes dienen.

Weitere Vorteile ergeben sich aus der Beschreibung und der beigefügten Zeichnung.

Ausführungsbeispiele der Erfindung werden unter Bezug auf die beigefügten Zeichnungen in der nachfolgenden Beschreibung näher erläutert. Es zeigen:
- Fig. 1: eine prinzipielle Skizze einer Vorrichtung, mit der das neue Verfahren durchgeführt wird;
- Fig. 2: eine prinzipielle Wachstumskurve, wie sie mit dem neuen Verfahren aufgenommen und extrapoliert werden kann;
- Fig. 3: für das blockstarre Zeit-Frequenz-Pulsverschränkungsverfahren mit fester Pulspaaranordnung die Einhüllenden für vier verschiedene f2-Anregungspulse mit den Frequenzen 1,5; 4; 2; 3 kHz;
- Fig. 3b: für das blockstarre Zeit-Frequenz-Pulsverschränkungsverfahren mit fester Pulsanordnung die Einhüllenden für vier verschiedene Pulspaare mit jeweils vier verschiedenen Anregungsfrequenzen f2 und f1 mit den Frequenzen f1 1,25; 1,67; 2,5; 3,33 kHz; und den Frequenzen f2 1,5; 2; 3; 4 kHz.
- Fig. 4: für das blockstarre Zeit-Frequenz-Pulsverschränkungsverfahren mit fester Pulspaaranordnung die Aufteilung eines Panels von vier f2-Pulspaaren auf vier Slots in einem Block;
- Fig. 5: für das blockstarre Zeit-Frequenz-Pulsverschränkungsverfahren mit fester Pulspaaranordnung die Aufteilung von insgesamt sieben f2-Pulspaaren auf zwei Panel und zwei Blöcke mit je vier Slots;
- Fig. 6: für das blockflexible Zeit-Frequenz-Pulsverschränkungsverfahren mit fester Pulspaaranordnung die Aufteilung von insgesamt sieben f2-Pulspaaren auf drei Panel und drei Blöcke mit je vier Slots;
- Fig. 7: für das blockflexible Zeit-Frequenz-Pulsverschränkungsverfahren mit freier Pulspaaranordnung die Aufteilung von insgesamt sieben f2-Pulspaaren auf vier Panel und vier Blöcke mit variabler Anzahl von Slots, unter der vereinfachten Annahme von einheitlichen Messzeiten für ausgewählte f2-Pulspaare; und
- Fig. 8: für das blockflexible Zeit-Frequenz-Pulsverschränkungsverfahren mit freier Pulspaaranordnung die Aufteilung von insgesamt sieben f2-Pulspaaren auf sechs Panel und sechs Blöcke mit variabler Anzahl von Slots, ohne vereinfachte Annahme von einheitlichen Messzeiten für ausgewählte f2-Pulspaare.

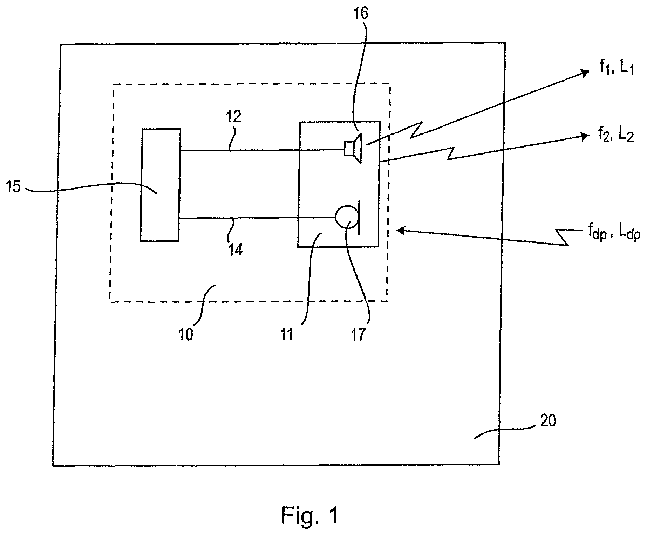

Eine erfindungsgemäß einzusetzende Vorrichtung 10 ist schematisch in Fig. 1 gezeigt, sie umfasst typischer Weise zumindest eine Ohrsonde 11, die per Kabel 12, 14 mit einer Soundkarte oder einer anderen A/D-Karte in einer Rechnereinheit 15, hier einem Computer verbunden ist.

Gemäß der eingangs erwähnten DE 199 05 743 A1 kann zwischen einem zwei Ohrsonden tragenden Kopfhörer und dem Computer eine Funkstrecke vorgesehen sein, um Störgeräusche zu vermeiden, die durch das Kabel verursacht werden könnten. Wegen der beiden Ohrsonden kann hier binaural gemessen werden.

Jede Ohrsonde 11 enthält mindestens einen hochlinearen Lautsprecher 16, mit dem die beiden Anregungssignale f1, L1 und f2, L2 abgeben werden, und mindestens ein Mikrophon 17, mit dem die DPOAE, also deren Schalldruckpegel Ldp bei einer mit Hilfe von f2 eingestellten Frequenz fdp = 2 f1 - f2 gemessen werden. Häufig enthält jede Ohrsonde 11 jedoch zwei Lautsprecher 16, damit bei der Stimulation mit den beiden Tönen f1, f2 keine technischen Distorsionen entstehen, die von den physiologischen DPOAE schwer zu trennen sind.

Der Computer kann extern vorgesehen oder in die Ohrsonde 11 integriert sein. Er ist dazu ausgelegt, die erfindungsgemäß verwendeten Pulspaare (f1, f2) zu generieren und während der Messung adaptiv anzupassen. Er kann die Pulspaare (f1, f2) und gemessenen DPOAE (Ldp, fdp) für eine spätere Analyse speichern und zum Auslesen bereitstellen.

Alternativ kann der Computer die Analysen auch in Echtzeit durchführen.

Das Ergebnis der Messung sind sogenannte Wachstumskurven für die ausgewählten Anregungsfrequenzen f2, aus denen der Computer 15 dann die jeweiligen Schwellwerte bestimmt, die eine objektive Bewertung des Hörvermögens darstellen und für unterschiedliche Zwecke verwendet werden können. Wichtig ist dabei, dass das neue Verfahren eine sehr schnelle Bestimmung der Wachstumskurven ermöglicht, was die Akzeptanz des Verfahrens befördert.

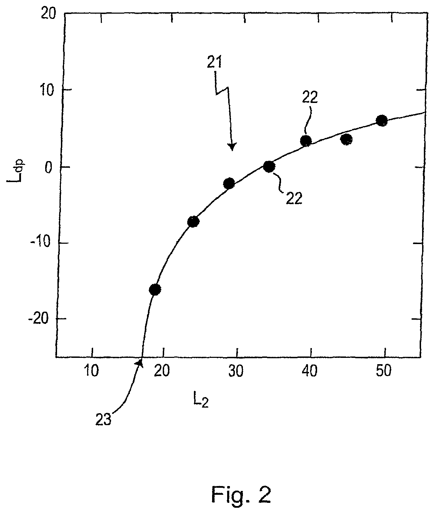

Eine prinzipielle Wachstumskurve 21 ist in Fig. 2 gezeigt. Über dem Schallpegel L2 ist der gemessene Pegel Ldp der DPOAE für eine Anregungsfrequenz f2 aufgetragen. Aus den sieben Messwerten 22 für die DPOAE ist der Schwellwert 23 extrapoliert.

Zur Anpassung der vom Computer ausgegebenen Signale f1, f2 an den in der Ohrsonde 11 eingebauten Lautsprecher 16 können ein Endverstärker und/oder eine Impedanzanpassung und Frequenzgangkorrektur vorgesehen sein, was in Form einer passiven oder aktiven elektronischen Schaltung realisiert wird.

Zur Anpassung der vom Mikrophon 17 erzeugten elektrischen Signale an die Computerschnittstelle können ein Vorverstärker und eine Frequenzgangkorrektur vorgesehen sein, was ebenfalls in Form einer passiven oder aktiven elektronischen Schaltung realisiert wird.

Befinden sich nicht alle beschriebenen Verarbeitungsstufen in der Ohrsonde 11, so ist entweder eine Kabelverbindung oder eine drahtlose Verbindung vorgesehen. Befinden sich alle Verarbeitungsstufen in der Ohrsonde 11, ist eine drahtlose Verbindung entweder für die Übergabe der Messdaten nach der Messung, oder in Echtzeit an ein Wiedergabegerät vorgesehen.

Die Daten können zudem als klinische Daten in klinischen Informationssystemen gespeichert werden.

Die insoweit beschriebenen Vorrichtungen 10 werden zur Untersuchung des Hörvermögens von Kinderärzten, in HNO-Kliniken, von HNO-Ärzten, von Hörgeräteakustikern und von Patienten zuhause eingesetzt. Sie können aber auch direkt in Hörgeräte integriert werden, um diese sozusagen im Betrieb an ein verändertes Hörvermögen des Trägers anzupassen.

In Fig. 1 ist ein Hörgerät 20 schematisch angedeutet, in dem die Vorrichtung 10 angeordnet ist.

Das mit diesen Vorrichtungen durchzuführende Verfahren wurde eingangs in seinen einzelnen notwendigen und bevorzugten Schritten beschrieben. Nachstehend erfolgt mit teilweise leicht abgewandelter Nomenklatur eine Gesamtdarstellung für bevorzugte Ausführungsbeispiele.

### 1. Einleitung

Ein zeitoptimales Verfahren für die Messung von DPOAE-Wachstumsfunktionen muss unterschiedliche Ebenen der Messwertaufnahme und -analyse berücksichtigen, die sich wie folgt unterteilen lassen:

### 1.1) Messung eines einzelnen DPOAE

Dies erfordert die Festlegung eines mindestens zu erreichenden Rauschabstands, die Festlegung, wie Signal und Rauschen berechnet werden soll, ein geeignetes Artefaktunterdrückungsverfahren, und in der Regel eine maximale Messzeit, ab welcher die Messung als erfolglos abgebrochen wird.

### 1.2) Schwellwertannäherung

In der Messung von Wachstumsfunktionen stellt sich die Frage, bei welchen Anregungspegeln, in welcher Reihenfolge, und wie viele Einzelmesswerte aufgenommen werden sollen. Im Fall des Extrapolationsverfahrens hat sich gezeigt, dass häufig nur die drei schwellennächsten Punkte einer Wachstumsfunktion zur genauen Schätzung der extrapolierten Schwelle beitragen, bzw., dass die Schätzgenauigkeit zunimmt, wenn andere Punkte weggelassen werden. Daraus resultiert der Gedanke, gezielt diese drei Punkte zu messen, um den Zeitaufwand für die übrigen Punkte einzusparen.

Wie sich zeigen wird, ist der schwellennächste Messpunkt am kritischsten. Wird sein Anregungspegel zu gering gewählt, muss entweder übertrieben lange gemittelt werden, oder er kann sogar nach Ablauf der maximalen Einzelwertmesszeit nicht mit der geforderten Sicherheit bestimmt werden. Wird er zu hoch gewählt, steigt der Extrapolationsfehler, da der letzte Messpunkt weiter von der zu schätzenden Schwelle entfernt ist.

Es wird daher ein Verfahren bereitgestellt, das aus einem ersten validen Einzelmesswert, der möglichst der dritte und schwellenfernste Messpunkt sein soll, die beiden folgenden Messpunkte nach optimalen Kriterien festlegt, wobei mit jedem hinzu kommenden Messpunkt die Berechnung des optimalen nächsten Anregungspegels adaptiv verfeinert werden soll.

Gesondert betrachtet wird die Frage, wie der erste und schwellenfernste Einzelmesswert angeregt werden soll. Hier geht es gewissermaßen um eine globale Strategie, die je nach Anwendung unterschiedlich ausfallen kann.

Generell ist es sinnvoll, den ersten Messwert bei etwa L2 = 45 dB SPL anzuregen, da erfahrungsgemäß oberhalb dieses Wertes Wachstumsfunktionen bei Normalhörenden bereits saturieren, d.h. Werte erzeugen, die für eine optimale Schwellenschätzung nicht hilfreich sind. Misst man bspw. Neugeborene im Rahmen eines Siebtests, wäre dies eine gute Wahl, weil die allermeisten Neugeborenen normalhörend sind und damit die mittlere Messzeit für den Siebtest voraussichtlich minimiert werden würde, da im Großteil der Population jeweils nur drei Einzelmesswerte (pro Frequenz) erhoben werden müssen, und nur selten der erste schwellenfernste Messpunkt erfolglos versucht wird.

In einer Anwendung für Patienten, die mit einem Hörverlust die Klinik aufsuchen, sind Wachstumsfunktionen, die einer normalhörenden Population entsprechen, viel seltener, während die Zahl der Fälle, wo mit L2 = 45 dB SPL die maximale Messzeit für einen Einzelmesswert verbraucht wird, ohne einen gültigen Wert zu erhalten, bereits beträchtlich sein kann.

In diesen Bereich sollen auch Verfahren gezählt werden, die eine Wachstumsfunktion neu positionieren, wenn keine optimale Anregung vorliegt, und solche, bei denen bei Multifrequenzmessungen, also Pulsverschränkungsverfahren mit mehreren Frequenzen, Wachstumsfunktionen abhängig von Ergebnissen bei anderen Frequenzen justiert werden.

### 1.3) Zeitliches Pulsverschränkungsverfahren

In der Regel soll der Zustand des Innenohrs nicht nur bei einer Frequenz erfasst werden, sondern bei einer geeigneten Anzahl von Frequenzen, typisch in Oktav- oder Halboktavschritten im Bereich 1kHz ≤ f2 ≤ 8kHz. Dies bedeutet, dass mehrere Wachstumsfunktionen gemessen werden müssen.

Gepulste DPOAE haben gegenüber kontinuierlichen DPOAE zunächst den gravierenden Nachteil, dass die Messung bei einer Frequenz-Schallpegelkombination generell eine geringe Zeitauslastung ("duty factor") und damit ein entsprechend geringeren Rauschabstand bei derselben Messzeit aufweist.

Dieser Nachteil wird jedoch erfindungsgemäß erheblich reduziert, indem mehrere Messungen im Zeit-Frequenzraum miteinander verschränkt werden, d.h. zeitversetzt abwechselnd präsentiert werden. So können innerhalb eines hinreichend oft wiederholten Blocks zeitversetzt bspw. 7 Frequenzen stimuliert und analysiert werden.

Die einfachste Realisierung ist eine feste Anordnung von Anregungspulsen innerhalb eines Blocks; dies wird blockstarres Verfahren genannt. Bei der Anordnung der Pulspaare innerhalb eines Blocks muss die Frequenz- und Zeitabfolge so gewählt werden, dass die Signale gegenseitig nur in vernachlässigbarem Umfang interferieren.

Dieses Verfahren funktioniert nahezu optimal, insbesondere wenn der Rauschhintergrund und das Auftreten von Artefakten bei allen verwendeten Frequenzen ähnlich sind.

Eine weitere Optimierung wird erreicht, wenn die stimulierenden Pulspaare nicht blockstarr, sondern blockflexibel angeordnet werden. Dies führt zu einer zusätzlichen Zeitersparnis, wenn die Messung bei einer Frequenz-Pegelkombination bereits einen ausreichenden Rauschabstand erreicht hat, während eine andere noch deutlich mehr Zeit erfordert.

In diesem Fall werden im blockflexiblen Pulsverschränkungsverfahren vor Ablauf einer der im Block momentan vorkommenden Einzelmessungen ein oder mehrere Pulspaare, die bereits den geforderten Rauschabstand erreicht haben, durch solche mit anderer Frequenz oder anderem Pegel ersetzt, die sich noch in der Messung befinden.

Hierfür wird ein Regelsatz bereitgestellt, der einen Mindestabstand im Zeit-Frequenz-Pegelraum definiert.
Im Einzelnen:

### 2.1) Messung eines einzelnen DPOAE

Geeignete Verfahren zur Bestimmung von Kriterien für die Einbeziehung von Messwerten - hier der DPOAE - in die Mittelwertbildung, und zur Berechnung eines Mittelwertes, der einen vorgegebenen Rauschabstand (SNR) aufweist, sind aus dem Stand der Technik hinreichend bekannt; siehe beispielsweise Müller und Specht, "Sorted averaging - principle and application to auditory brainstem responses", in Scand. Audiol. 1999, 28: 145-9.

Die Erfinder haben jedoch festgestellt, dass die Abklingzeit von Pulsantworten ein Problem darstellen kann für den Ansatz, durch Kürzung der Blocklänge die Messzeit zu reduzieren.

In typischen Messungen im Frequenzbereich um f2 = 2 kHz reicht eine Blocklänge von T = 70 ms aus, um sicherzustellen, dass die Pulsantwort innerhalb eines Blocks soweit abgeklungen ist, dass sie im folgenden Block durch Interferenz mit der Pulsantwort auf den erneuten Stimulus durch ein weiteres Pulspaar keine nennenswerten Messfehler erzeugt.

Dies gilt allerdings nicht, wenn sich die Frequenz der gesuchten DPOAE, i.d.R. fdp = 2f1 - f2, in der Nähe einer spontanen otoakustischen Emission (SOAE) befindet.

Um aus dieser Problematik resultierende Messfehler zu begrenzen, bedienen sich die Erfinder verschiedener Vorgehensweisen:
A) Anpassung der Blocklänge an die Abklingzeit des Pulses unter einen gewissen Pegel. Dieses Verfahren hat den Nachteil, dass u.U. eine erhebliche Verlängerung der Messzeit in Kauf genommen werden muss, und dass bereits eine gewisse Mittelung mit dazugehörigem Zeitaufwand nötig ist, um die Abklingzeit zu bestimmen. Das Verfahren ist daher nur dann sinnvoll, wenn die Messung bei einer genau einzuhaltenden Frequenz gefordert wird.
B) In der klinischen Praxis ist die Messung bei einer genau definierten Frequenz jedoch nicht erforderlich. Ein ausreichendes Bild über den Zustand der Cochlea entsteht nach allgemeiner Ansicht durch Messung bei Oktavfrequenzen, bei höherem Anspruch bei Halboktavfrequenzen, also beispielsweise bei f2 = 0,75; 1; 1, 5; 2; 3; 4; 6; 8 kHz.

In derartigen Anwendungen dürfte die Messung bei einer Frequenz f2, die um 50 bis 150Hz neben den Sollfrequenzen liegt, vollkommen ausreichen. Daher soll die Existenz von SOAE geprüft werden, und zwar a) entweder in einer a priori-Messung, deren Auswertung dazu genutzt wird, die tatsächliche Frequenz f2 des Stimulus zu verschieben, um einen ausreichenden Sicherheitsabstand zwischen SOAE und fdp zu erhalten, oder b) bei der Messung der DPOAE bei ihren Sollfrequenzen f2 das Problem einer zu hohen Abklingzeit zu erkennen, und zwar durch einen Algorithmus, der während der Messung mitläuft, und dann im Bedarfsfall die Messung mit verschobenen Stimulusfrequenzen f2 zu wiederholen.

Die Wahl zwischen diesen beiden Varianten kann durch die Wahrscheinlichkeit des Auftretens von SOAE in der gegebenen Anwendung entschieden werden. Sind bei einer Anwendung bei stärker Hörgeschädigten nur äußerst selten SOAE zu erwarten, wird man den Zeitaufwand für die Messung von SOAE sparen wollen (z.B. Hörscreening bei älteren Erwachsenen), bei Neugeborenen hingegen sind SOAE fast sicher existent und damit die Wahrscheinlichkeit, dass sie die DPOAE-Messung verfälschen, höher.

Zu A) Eine SOAE-Messung dauert typischerweise 40s, um einen Rauschhintergrund von -30dB SPL bei f = 2kHz zu erreichen. Der erforderliche Frequenzversatz kann in Abhängigkeit von der Stärke der gefundenen SOAE eingestellt werden.

Zu B) Hier bietet sich an, den Algorithmus der optimierten Mittelung zu verwenden. Er sagt vorher, dass das Rauschen bei Ausschluss von Blöcken mit ungewöhnlich hohem Rauschen einem klaren Gesetz folgt. Tut es das nicht, oder wird nach einer gewissen Zeit praktisch jeder Block ausgeschlossen, ist dies ein Zeichen dafür, dass das gewählte Rauschmaß nicht mehr der Annahme eines zufälligen, unkorrelierten Prozesses folgt.

Bezieht sich das Rauschmaß bspw. auf die Amplitude der Einhüllenden des Zeitsignals unmittelbar bevor eine erneute Pulsantwort erwartet wird, kann für eine gewisse Anzahl von Blöcken der Mittelungsprozess ordnungsgemäß funktionieren. Sobald aber die Rauschamplitude auf den Wert der abgeklungenen Pulsantwort auf den vorhergehenden Stimuluspuls abgesunken ist, wird sich die Amplitude durch Mittelung nicht mehr verringern, wenn die abklingende Pulsantwort hochreproduzierbar ist und damit eine hohe Korrelation aufweist.

Ein Schema für den Messstart einer Wachstumsfunktion sieht vor, dass die Messung beginnt mit einem Startpegel L2 für den zweiten Primärton bei f2. Der Pegel L1 für den ersten Primärton f1 wird nach einer Normkurve für die betreffende Frequenz ermittelt, z.B. nach der Pegelschere, wie sie beschrieben ist von P. Kummer et al., "The level and growth behavior of the 2f1-f2 distortion product otoacoustic emission and its relationship to auditory sensitivity in normal hearing and cochlear hearing loss", in J.Acoust.Soc.Am., 103(6):3431-3444, 06 1998.

Kann kein DPOAE gemessen werden, wird der Pegel L2 des zweiten Primärtons um ΔL2 erhöht, bis der maximal erzeugbare Primärtonpegel erreicht wird. Liegt auch dann kein DPOAE vor, wird noch eine Optimierung des ersten Primärtonpegels L1 versucht.

Wird ein DPOAE gemessen, schließt sich das nachstehend beschriebene Verfahren der Schwellenannäherung an.

### 2.2) Schwellwertannäherung

### 2.2.1 Festlegen des ersten Messpunktes

Da bei Normalhörenden die Erfahrung besteht, dass die DPOAE-Wachstumsfunktionen zum Teil relativ frühzeitig saturieren, soll der erste Messwert so aufgenommen werden, dass er möglichst dem höchsten Anregungspegel entspricht, bei dem i.d.R. noch keine Saturation zu erwarten ist. Der so gemessene Punkt wäre im Fall eines Normalhörenden dann noch verwertbar und damit ohne Zeitverlust gemessen. Er würde zweckmäßigerweise der schwellenfernste Punkt der Wachstumsfunktion sein.

Nach bisherigen Erfahrungen bietet sich ein Anregungspegel von L2 = 45 dB SPL an. Wird dieser Punkt zu niedrig gewählt, steigt die Anzahl der Fälle, in denen die maximal zulässige Messzeit erreicht wird, ohne dass ein verwertbarer Einzelmesswert erhalten wird.

Hier kann wahlweise die Strategie folgendermaßen variiert werden: Die maximal zulässige Messzeit wird für den ersten Messpunkt reduziert. Dies führt dazu, dass weniger Zeit verloren wird, wenn die DPOAE-Schwelle oberhalb L2 liegt. Liegt sie nur knapp darunter, kann in einem späteren Schritt, wenn L2 mit längerer Messzeit gemessen werden soll, die Erfassung desselben Messpunktes wieder aufgenommen werden. Die letztere Methode nutzt den Vorteil der vergleichsweise schnell gewonnenen Informationen der oberen Punkte in der Wachstumsfunktion, um den Pegel des untersten Punktes der Wachstumsfunktion zeitoptimal einstellen zu können, womit die höchste Zeiteffizienz erreicht wird.

### 2.2.2 Festlegen des zweiten Messpunktes

Im Weiteren wird von der Messung von drei Pulspaaren für eine gegebene f2, also von drei verschiedenen Schallpegeln L2 für jede zweite Anregungsfrequenz f2 ausgegangen. Dies entspricht der minimalen Anzahl von Punkten, auf die das Extrapolationsverfahren mit Qualitätskontrolle (Bestimmung des Korrelationskoeffizienten und der Standardabweichung der geschätzten Schwelle) angewandt werden kann.

Die Messungen bei Normalhörenden zeigen eine hohe Korrelation zwischen der Steigung der Wachstumsfunktion und der Amplitude des DPOAE bei mittlerem Anregungspegel, etwa L2 = 45dB SPL. Es ist daher zweckmäßig, den zweiten Anregungspegel so zu wählen, dass er in der Mitte zwischen dem ersten und dem dritten Anregungspegel liegen wird. Hierzu wird die Steigung der Wachstumsfunktion geschätzt, und zwar anhand von Populationsmittelwerten, siehe Dalhoff et a., 2013, Hear. Res. Bd. 296, Tab. 2, Seite 77, die am besten frequenzabhängig ermittelt werden.

Mit Hilfe der Steigung kann dann der zeitoptimale Anregungspegel L2 für den letzten, schwellennächsten Messpunkt festgelegt werden. Hierfür wird in Abschnitt 2.2.3 ein Verfahren angegeben, bei dem dann in Gleichung (2) für m der oben erwähnte Populationsmittelwert eingesetzt wird. Anhand des ersten (schwellfernen) Messpunktes und Populationsdaten wird dann der schwellnächste (dritten) Messpunkt geschätzt. Der zweite Messpunkt wird dann in die Mitte zwischen den ersten und dritten Messpunkt gelegt.

### 2.2.3 Festlegen des letzten Messpunktes bei niedrigstem Anregungspegel L2

Es wird davon ausgegangen, dass bereits zwei oder mehr Messpunkte einer Wachstumsfunktion bei höheren Anregungspegeln L2 vorliegen. Gemäß Abschnitt 2.2.2 liegen beispielsweise die beiden oberen Messpunkte vor, so dass nun der dritte (schwellnächste) Messpunkt erneut bestimmt werden kann, jedoch jetzt mit der individuell bestimmten Steigung.

Dazu soll die Aufgabe gelöst werden, wie der letzte und niedrigste Anregungspegel L2 so zu wählen sei, dass mit dem geringstmöglichen Zeitaufwand ein minimaler Schätzfehler in der abschließenden Extrapolation auf die Schwelle der Wachstumsfunktion erhalten wird.

Dazu müssen zwei Fragen beantwortet werden. Erstens: Welchen Einfluss hat ein aufgrund begrenzter Mittelungszeit erhaltener Messfehler des letzten, schwellennächsten Messpunktes auf die Regression und damit auf den Schätzfehler der extrapolierten Schwelle? Hier ist prinzipiell klar, dass ein Messpunkt nahe an der Schwelle bei gleichem Messfehler zu einem geringeren Extrapolationsfehler führen wird. Zweitens: Der letzte Punkt der Wachstumsfunktion muss allerdings mit einem gewissen Rauschabstand gemessen werden, damit er als gültig angesehen werden kann, und dies bedeutet, dass, je näher er am Schwellwert der Wachstumsfunktion gewählt wird, die erforderliche Mittelungszeit um so mehr ansteigen wird. Aus diesen beiden Überlegungen wird sich ein eindeutiges Optimalitätskriterium ergeben.

Die Punkte der Wachstumsfunktion seien vom niedrigsten zum höchsten Anregungspegel hin nummeriert, also entgegen der zeitlichen Messabfolge. Folglich suchen wir den Punkt *P*₁ mit dem Anregungspegel *x*₁, während die Punkte *P*₂, ..., *Pₙ* bereits vorliegen. Aufgrund der vorliegenden Punkte konnte bereits eine vorläufige Regressionsgerade (bzw., bei *n* = 3 eine Verbindungsgerade) ermittelt werden, mit *y*(*x*) = *m*_{*e,n* - 1}*x + b*_{*e,n -* 1}, wobei e für *estimated* stehen soll, und *n* - 1 die Anzahl der zur Schätzung herangezogenen Punkte bedeutet.

Im Folgenden wird davon ausgegangen, dass *y*(*x*) die tatsächliche Wachstumsfunktion ohne jeglichen Fehler beschreibt, während der neu hinzukommende Messpunkt aufgrund endlicher Mittelungszeit einen Messfehler *N*₁ aufweist, so dass der gemessene (bzw. aus der Messung geschätzte) Wert eine Amplitude von *y*_{1*,e*} = *y*₁ + *N*₁ aufweisen wird, wenn *y*₁ die wahre Amplitude ist.

Die Steigung der Regressionsgeraden, die aus der fehlerbehafteten Messung von *P*₁ hervorgeht, ergibt sich aus: ${m}_{e} = \frac{\overline{{xy}_{e}} - \overline{{xy}_{e}}}{\overline{{x}^{2}} - {\overline{x}}^{2}}$

Dabei bedeutet der Strich über den Variablen Mittelwertbildung. Damit ist $Δ m = {m}_{e} - m = \frac{\overline{{xy}_{e}} - \overline{{xy}_{e}}}{\overline{{x}^{2}} - {\overline{x}}^{2}} - m$

Der Achsenabschnitt berechnet sich zu: ${b}_{e} = \overline{{y}_{e}} - {m}_{e} \overline{x}$ und Δ*b* = *bₑ* - b wird $Δ b = \frac{{N}_{1}}{n} - Δ m \overline{x}$

Den aufgrund des Messfehlers entstehenden Extrapolationsfehler Δ*x_{edpt}* finden wir mit der Quotientenregel, sofern der Fehler nicht zu groß ist: $Δ {x}_{edpt} = \frac{b Δ m - m Δ b}{{m}^{2}}$

Es folgt: $nm Δ {x}_{edpt} = {N}_{1} \frac{\left({x}_{1}-\overline{x}\right) \left(\overline{x}-{x}_{edpt}\right) - β}{β}$ mit der Abkürzung $β = \overline{{x}^{2}} - {\overline{x}}^{2}$

Nach Separation des gesuchten Punktes x₁ aus den Mittelwerten finden wir ${N}_{1 , reg} = \frac{{nmΔx}_{edpt} \left({{x}_{1}}^{2}\left(\frac{n - 1}{n}\right)-\frac{2 {γx}_{1}}{n}+δ-\frac{{γ}^{2}}{n}\right)}{{x}_{1} \left({x}_{edpt}\left(1-n\right)+γ\right) + {γx}_{edpt} + δ}$ mit $γ = {\sum }_{2}^{n} {x}_{i}$ und $δ = {{\sum }_{2}^{n} {x}_{i}}^{2}$.

Dabei bezeichnet der Zusatz im Index *N*_{1,}*_{reg},* dass hier das hinnehmbare Rauschen für das Regressionskriterium gemeint ist. Aus dieser Gleichung berechnet sich also das zulässige Rauschen am Punkt *P*₁, wenn ein maximaler Extrapolationsfehler Δ*x_{edpt}* vorgegeben ist.

Für das Rauschabstandskriterium lautet die Forderung: ${N}_{1 , snr} = \frac{m \left({x}_{1}-{x}_{edpt}\right)}{SNR}$

Dabei ist der Rauschabstand *SNR* als lineares Verhältnis der Amplituden zu verstehen. Aus den beiden Anforderungen an das Rauschen definiert in den Gleichungen (8) und (9) kann die erforderliche Mittelungszeit bestimmt werden, wenn eine Rauschamplitudendichte *Ṅ* bekannt ist: $τ = {\left(\frac{\dot{N}}{{N}_{1}}\right)}^{2}$

Da beide Bedingungen gleichzeitig erfüllt sein sollen, ist die jeweils längere Messzeit einzuhalten. Gleichsetzen der beiden Rauschkriterien führt zu einer quadratischen Gleichung mit folgender Lösung: ${x}_{1} = - \frac{p}{2} - \sqrt{\frac{{p}^{2}}{4} - q}$ mit $p = - \frac{\left(2Δ{x}_{edpt}SNRγ+δ-{x}_{edpt}^{2}\left(1-n\right)\right)}{α}$ $q = \frac{\left(Δ{x}_{edpt}SNR\left(nδ-γ\right)+{x}_{edpt}\left({γx}_{edpt}+δ\right)\right)}{α}$ $α = \left(Δ{x}_{edpt}SNR+{x}_{edpt}\right) \left(n-1\right) - γ$

In der Praxis kann es notwendig sein, von der Empfehlung abzuweichen. So ist bekannt, dass bei Normalhörenden die Wachstumsfunktion unterhalb eines Pegels von etwas *L*₂ = 25 *dB SPL* nicht mehr dem idealen linearen Verlauf in der semilogarithmischen Darstellung folgt, da es genaugenommen gar keine Distorsionsproduktschwelle gibt. Wird aufgrund des oben erläuterten Verfahrens ein Anregungspegel in diesem Bereich empfohlen, wird die Schätzgenauigkeit systematisch verschlechtert. Hier kann bspw. ein minimaler Wert für ${L}_{2}^{\left(1\right)}$ festgelegt werden.

Alternativ kann in einem vereinfachten Verfahren auch ein Satz von L2-Schallpegeln im Computer hinterlegt sein, der für jede Anregungsfrequenz f2 verwendet wird. Es ist auch vorgesehen, für jede Anregungsfrequenz f2 einen gesonderten Satz von L2-Schallpegeln zu hinterlegen.

### 2.3) Zeitliches Pulsverschränkungsverfahren

### 2.3.1 Zeit-Frequenz-Pulsverschränkungsverfahren

### 2.3.1.1 Blockstarres Zeit-Frequenz-Pulsverschränkungsverfahren

Wir gehen davon aus, dass in einem Block ein Panel mit mehreren Pulspaare unterschiedlicher Frequenzen f1, f2 und auch Pegel L1, L2 präsentiert werden, und trotzdem aus bspw. 4 solchen Blöcken jeweils im PTPV-Verfahren (siehe beispielsweise Zelle et al., "Extraction of otoacoustic distortion product sources using pulse basis functions", in J.Acoust.Soc.Am., 134(1):EL64-EL69, 07 20139) sogenannte Ensembles gebildet werden, die die Extraktion des Zeitsignals einer gewünschten Distorsions-komponente, bspw. bei fdp = 2f1 - f2 ermöglichen.

Wir nennen diesen Präsentationsmodus blockstarr, wenn die Abfolge der Pulse innerhalb eines Blocks vorgegeben bzw. während der Messung unveränderlich ist.

Ein möglicher Ansatz ist es, in einem Block n Pulspaare unterschiedlicher Anregungsfrequenzen f2(i), i = 1, 2, 3, . . .n zeitversetzt anzuordnen. Die Blocklänge wird beispielsweise zu T (bzw. T_{B}) = 160 ms gewählt, die Startzeiten der Pulspaare werden gleichmäßig über den Block verteilt, in diesem Beispiel mit n = 4 und f2 = 1.5; 3; 2; 4 kHz. Das Hauptinteresse liegt dann auf der Extraktion der Distorsionskomponente bei fdp = 2f1-f2.

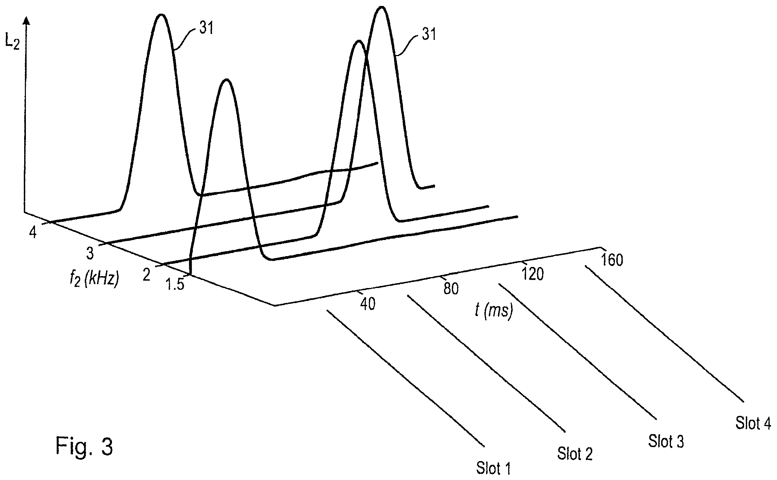

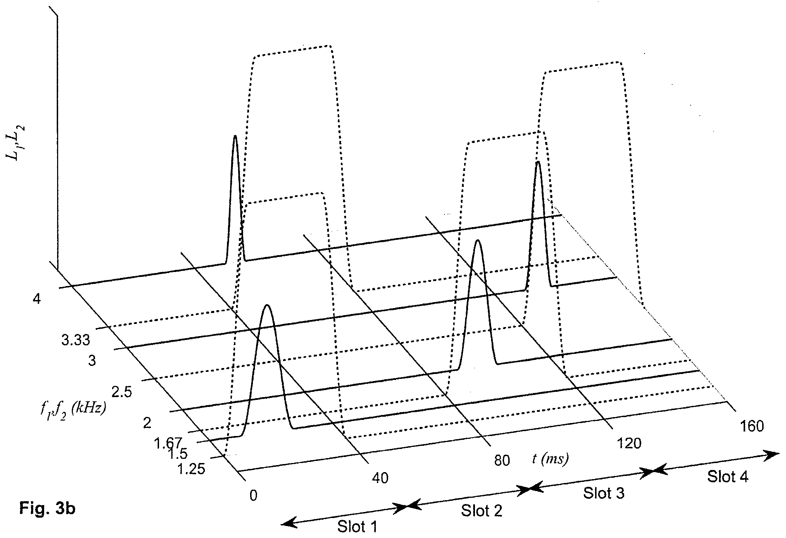

In Fig. 3 sind für das blockstarre Zeit-Frequenz-Pulsverschränkungsverfahren die Einhüllenden 31 für vier verschiedene f2-Anregungspulse mit den Frequenzen 1,5; 4; 2; 3 kHz dargestellt. Mit diesen Einhüllenden werden die Anregungstöne an- bzw. wieder abgeschaltet; die Pulsform ist in dieser Darstellung nur durch kosinusförmige Rampen ohne einen steady-state dargestellt. Fig. 3 zeigt sozusagen eine Momentaufnahme eines Messblocks zu einem Panel.

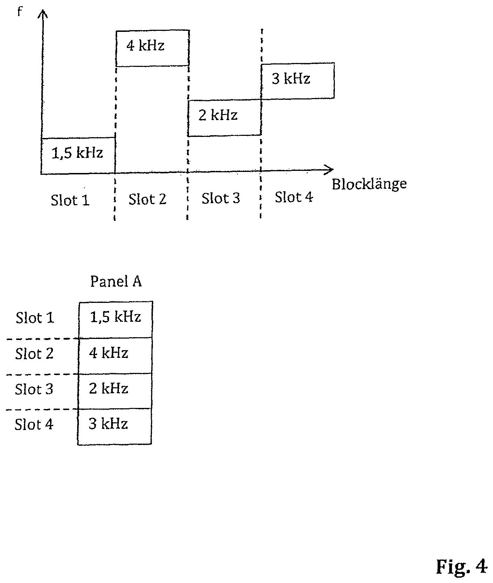

In Fig. 4 ist die Verteilung der in einem Panel A zusammengefassten vier Pulspaare auf die vier Slots des Blocks gezeigt.

In Fig. 3b sind für das blockstarre Zeit-Frequenz-Pulsverschränkungsverfahren die Einhüllenden 31 für vier verschiedene f2 Anregungsimpulse mit den Frequenzen 1,5; 4; 2; 3 kHz sowie die Einhüllenden 32 für die in den Pulspaaren korrespondierenden vier verschiedenen f1-Anregungspulse mit den Frequenzen 1,25; 3,33; 1,67; 2,5 kHz dargestellt. Fig. 3b stellt dabei einen Block mit vier Pulspaaren dar, die in vier Slots (Slot 1 bis Slot 4) von jeweils 40 ms Länge präsentiert werden. Wie ersichtlich, werden die Anregungsfrequenzen f1 der Pulspaare jeweils zu Beginn eines Slots angeschaltet und zum Ende eines Slots wieder abgeschaltet. Aufgrund der Slotlänge bzw. der Anschaltungslänge der Anregungspulse der f1-Frequenzen bilden diese, wie aus ihren Einhüllenden ersichtlich, jeweils Plateaus aus. Die vier verschiedenen f2-Anregungsfrequenzen, welche in vier Anregungspulsen innerhalb der vier Slots präsentiert werden, werden jeweils erst eingeschaltet, nachdem die f1-Anregungspulse eingeschaltet worden sind. Ferner werden die f1-Anregungspulse erst abgeschaltet, nachdem die f2-Anregungspulse ausgeschaltet und bereits abgeklungen sind. Die An- und Abschaltprozesse der einzelnen Anregungspulse f1, f2 sind durch kosinusförmige Rampen dargestellt. Bei den f1-Pulsen bilden die Plateaus hingegen ein steady-state, welches zwischen den kosinusförmigen Rampen des Ein- und Abschaltvorgangs liegt. Die Blockzeit T_{B} in dem dargestellten Block aus vier Pulspaaren f1, f2 beträgt 160 ms, welches der Summe der vier Slotzeiten T_{S} (4 x 40 ms) entspricht. Wie in den Paragraphen [0023] bis [0024] und [0050] dargestellt, erlaubt die Präsentation von kurzen f2-Pulsen (bzw. die Präsentation von kurzen Pulsen für mindestens eine der beiden Töne des Pulspaares) die Trennung zweier Signalkomponenten im Zeitsignal des Distorsionsproduktes, die bei der sonst üblichen kontinuierlichen Präsentation durch Interferenz zu verfälschten Meßergebnissen führen können.

Das Panel A der 4 f2-Pulspaare wird in einem Block von 160 ms präsentiert, in dem jedes f2-Pulspaar einen Slot von 40 ms einnimmt. Dieser Block wird solange wiederholt, bis für alle 4 gemessenen DPOAE ein vorgewählter Signal-Rausch-Abstand (hier auch als SNR bezeichnet) erreicht ist.

Diese Wahl der Frequenzen und ihre Anordnung sind aus zwei Gründen zweckmäßig:

### A) Maskierung und Interferenz

Frequenzen in Halboktavschritten, also hier für Slots i = 1; 3 und i = 2; 4, werden mit maximalem Abstand in dem Block angeordnet, da das Frequenzverhältnis f2/fdp etwa 1.5 beträgt. Dies führt dazu, dass prinzipiell zwei Störfälle dominieren - im vorliegenden Beispiel für i = 1;3:
I) Die Präsentation des f2(i=3)-Pulses kann auf der Cochlea die Antwort fdp(i=1) maskieren, wenn sie noch nicht ausreichend abgeklungen ist. Dies gilt allerdings nur für die zweite Quelle, die häufig nicht im Vordergrund des diagnostischen Verfahrens steht.
II) Die Präsentation des f2(i=3)-Pulses kann auf der Cochlea den f2(i=1)-Puls maskieren, da sie nur etwa eine kleine Terz auseinanderliegen. Hier wird auch der i.d.R. diagnostisch wichtigere primäre Beitrag zum DPOAE betroffen. Hier führt also der Zeitabstand von T/2 = 80 ms dazu, dass die störenden bzw. maskierenden Signalkomponenten i.d.R. auf ein akzeptables Maß abgeklungen sind.

### B) Zeitbedarf

In diesem blockstarren Verfahren muss so lange gemittelt werden, bis die Frequenz, die den schlechtesten Rauschabstand bzw. die höchste Artefaktrate liefert, den geforderten Mindestrauschabstand erreicht hat. Im vorliegenden Beispiel wird dies die Messung bei f2 = 1.5 kHz sein. Deren Rauschhintergrund ist aber typischerweise nicht mehr als 30% schlechter als bei den Frequenzen f2 = 2; 3; 4 kHz, so dass sich der Zeitverlust in Grenzen hält.

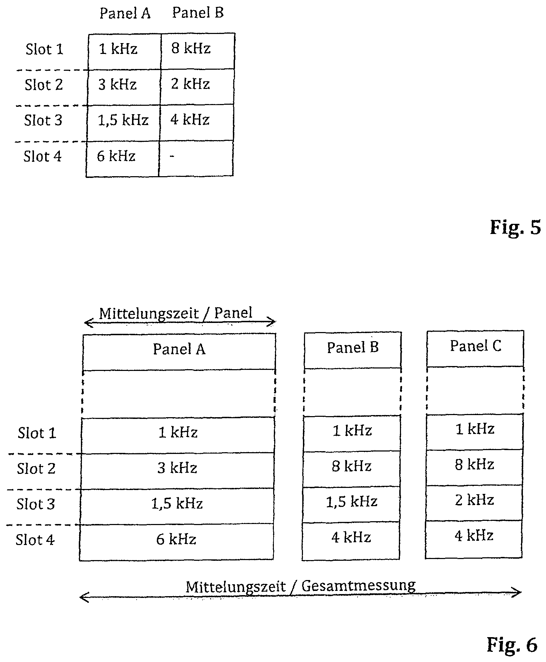

Fig. 5 zeigt die Aufteilung von sieben Anregungsfrequenzen f2 auf zwei Panel A und B in zwei Blöcke mit jeweils vier Slots zu 40 ms. Hier wird zuerst das Panel A abgearbeitet, also für jede f2 der Mittelwert des Schallpegels Ldp der zugehörigen DPOAE mit dem gewünschten SNR bestimmt.

Danach wird das Panel B in gleicher Weise abgearbeitet. Der Block mit Panel B hat hier auch 160 ms Blockdauer, er könnte jedoch auf 120 ms verkürzt werden, weil die Frequenzabstände zwischen den f2 und den fdp hinreichend groß sind.

### 2.3.1.2 Blockflexibles Zeit-Frequenz-Pulsverschränkungsverfahren mit fester Pulsanordnung

Soll ein Frequenzbereich überdeckt werden, in dem der Rauschhintergrund oder die Artefakthäufigkeit deutlich variiert, bzw. soll das Verfahren individuell bei jedem Patient optimal laufen, so muss von einer großen Variabilität hinsichtlich der erforderlichen Mittelungszeit für die jeweilige Frequenz f2 ausgegangen werden.

Eine Lösung für dieses Problem ist eine blockflexible Methode der Anordnung von Pulspaaren. Die verwendeten Frequenzen sind beispielsweise f2 = 1; 1.5; 2; 3; 4; 6; 8 kHz. Unter normalen Umständen wird die Messung bei f2 = 1kHz viermal so lange dauern wie bei f2 = 2; 3; 4 kHz, da der Rauschhintergrund doppelt so hoch ist, zumindest, wenn derselbe Rauschhintergrund erreicht werden soll.

Es gibt prinzipiell zwei Strategien, wie man auf diese Situation reagieren kann: Erstens kann man den höheren Rauschhintergrund in Kauf nehmen. Die Folge ist dann, dass bei niedrigen Frequenzen die Schätzgenauigkeit nicht so hoch ausfällt, da im Verlauf der Schwellenannäherung dieses Rauschen bei der Festlegung des schwellennächsten Punktes der Wachstumsfunktion berücksichtigt wird und ein höherer Extrapolationsfehler in Kauf genommen wird. Zweitens kann man versuchen, der Frequenz mit dem erhöhten Rauschhintergrund entsprechend mehr Messzeit zur Verfügung zu stellen.

Die Frequenzen werden daher auf drei Panel A, B und C aufgeteilt, die jeweils in einem Block mit 4 Slots präsentiert werden: in Slot 1 wird permanent, also für alle drei Panel das Pulspaar bei f2 = 1kHz präsentiert, in Slot 3 für 3/4 der Zeit das Pulspaar bei f2 = 1.5kHz, und für den Rest der Zeit das Pulspaar bei f2 = 2kHz; in Slot 2 für die erste Hälfte der Messzeit das Pulspaar bei f2 = 3kHz, für die zweite Hälfte das Pulspaar bei f2 = 8kHz, und in Slot 4 für die erste Hälfte der Messzeit das Pulspaar bei f2 = 6kHz, für die zweite Hälfte das Pulspaar bei f2 = 8kHz.

Fig. 6 zeigt die Aufteilung der sieben f2-Pulspaare auf insgesamt drei Panel A, B und C. Auch hier werden die Panel A, B und C nacheinander abgearbeitet. Im Panel A werden die Mittelwerte für die Ldp bei f2 = 3 kHz und f2 = 6kHz bereits mit hinreichendem SNR bestimmt. Im Panel B wird die Messung für f2 = 1 kHz fortgesetzt, für f2 = 1,5 kHz abgeschlossen, und für f2 = 4kHz und f2 = 8 kHz begonnen. Die Messzeit für Panel A ist größer als die für Panel B. Im Panel C wird die Messung für f2 = 1 kHz, f2 = 4kHz und f2 = 8 kHz abgeschlossen und für f2 = 2 kHz aufgenommen und beendet.

In einer derartigen Anordnung wird den unterschiedlichen Rausch- bzw. Artefaktbedingungen für die verschiedenen Anregungsfrequenzen Rechnung getragen; es wurde sichergestellt, dass nie die Präsentation einer f2(i + 1) eine halbe Oktave höher als f2(i) direkt auf letztere folgt, und zusätzlich wird in Slot 2 und 4 die Gesamtmesszeit asymmetrisch auf die drei Panel aufgeteilt werden, so dass die hohen Frequenzen, f2 = 6; 8 kHz, mehr Mittelungszeit zugeteilt bekommen, wenn bei ihnen der Rauschhintergrund wieder leicht ansteigt.

Hier wird der erste Anregungspegel zuerst abgearbeitet, worauf entsprechend dem Verfahren der Schwellenannäherung der zweite Anregungspegel in derselben Anordnung folgt etc.

Auch dieses blockflexible Verfahren kann suboptimal arbeiten, insbesondere, wenn die DPOAE deutlich unterschiedlich ausfallen, also höchstwahrscheinlich eine erhebliche Hörstörung vorliegt. Ein höheres Niveau des Pulsverschränkungsverfahrens kann daher nur mit freier, situationsangepasster Pulsanordnung erreicht werden.

### 2.3.1.3 Blockflexibles Zeit-Frequenz-Pulsverschränkungsverfahren mit freier Pulsanordnung

Hier werden der Messung von Wachstumsfunktionen nach dem Schwellenannäherungsverfahren Jobs zugewiesen. Werden DPOAE für sieben Frequenzen f2 in Halboktavschritten gemessen, werden also auch sieben Jobs abgearbeitet. Die Messungen werden zweckmäßigerweise mit möglichst ähnlichen Anregungspegeln L2 durchgeführt, damit Maskierungsprobleme reduziert werden. Also starten alle Jobs mit dem Pegel, der bei Normalhörenden der schwellenfernste sein wird.

Diese sieben Jobs werden jedoch nicht notwendigerweise parallel gestartet. Die Messung wird vielmehr mit Panel A begonnen. Die Panel B bis D sind noch nicht festgelegt, sie ergeben sich durch die automatisiert vorgenommene neue Belegung eines Slots nachdem die Rechnereinheit 15 festgestellt hat, dass ein entsprechendes DPOAE für ein in Panel A vermessenes Pulspaar ein hinreichendes SNR aufweist.

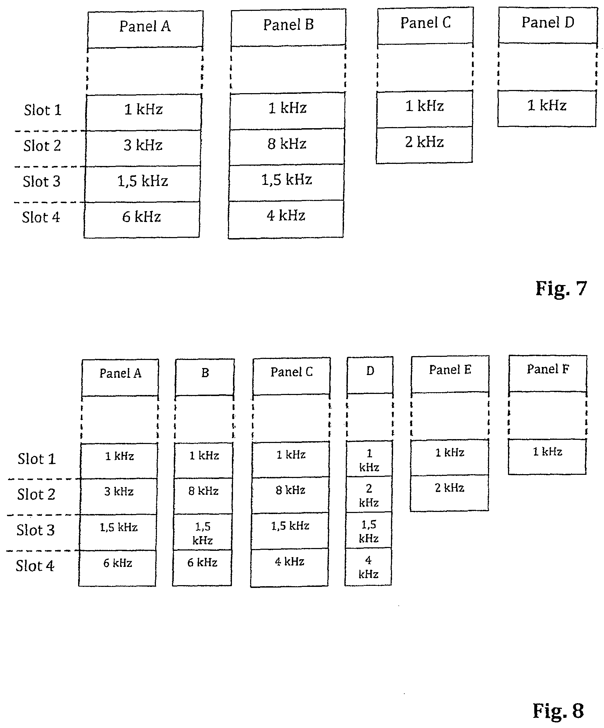

Die Fig. 7 und 8 stellen den Ablauf der Neubelegung beispielhaft dar. Die Jobs sind gemäß Fig. 7 auf vier Panel A, B, C und D mit bis zu vier Slots innerhalb eines Blocks verteilt. Die momentane Anordnung von Jobs - also Pulspaaren unterschiedlicher f2 - in den vier Slots wird als Panel bezeichnet und muss einem Satz von Kriterien entsprechen, die beispielsweise Maskierungseffekte berücksichtigen.

Für die Slots werden geschätzte Laufzeiten in eine Matrix eingetragen. Bei Start der Messung (Panel A) beruhen die Schätzwerte auf empirischen Daten der für die diagnostische Aufgabenstellung dominierenden Population. Während der Präsentation der einzelnen Blöcke werden alle Jobs verfolgt. Sobald ein Job sein Abbruchkriterium erreicht hat (ausreichendes SNR oder Erreichen der maximalen Präsentationszeit), werden die geschätzten Bearbeitungszeiten der Slots neu berechnet und der frei werdende Slot mit einem noch abzuarbeitenden Pulspaar neu belegt.

Im Beispiel besteht die Startaufstellung aus derselben Anordnung wie oben in 2.3.1.2 angegeben, wird jedoch im Folgenden variiert. Für die Startaufstellung wurde angenommen, dass Messungen bei f2 = 1kHz wegen doppeltem Rauschniveau die vierfache Zeit benötigen wie solche bei f2 ≥ 2 kHz, solche bei f2 = 1.5 kHz noch die doppelte Zeit.

Dann ist die gewählte Aufstellung annähernd optimal; theoretisch wird in Panel C Zeit verschenkt, da die Messung bei 2kHz vorzeitig beendet sein wird. Entscheidend ist der Wechsel zwischen Panel B und C: Hier wurde die Zahl der Slots auf zwei reduziert, da der notwendige Abstand von einer Oktave bei den beiden verbliebenen Frequenzen eingehalten wird, so dass pro Zeit doppelt so viele Pulse präsentiert werden können wie im 4-Slot-System. Alternativ kann theoretisch dieselbe Messzeit durch ein durchgängiges 2-Slot-System erhalten werden.

Wird nun nach Abarbeitung von Job 2 und 7 (f2 = 3 bzw. 8kHz) festgestellt, dass diese deutlich zeitiger oder die Jobs in den verbliebenen Slots verzögert ablaufen, wird geprüft, ob ein noch nicht bearbeiteter Job übernommen werden kann. Das kann in diesem Fall Job 3 (f2 = 2kHz) sein. Wird er allerdings abgearbeitet, bleibt am Ende nur noch Job 1 übrig, so dass voraussichtlich keine Zeit gespart wird. Sobald aber auch die Jobs in Slot 4 vorzeitig beendet wurden, kann früher in das 2-Slot-System umgeschaltet werden, und nach Abarbeiten von Job 3 (f2 = 2kHz) die Messung mit einer Einzelmessung bei 1 kHz beendet werden.

Während in Fig. 7 für das blockflexible Zeit-Frequenz-Pulsverschränkungsverfahren mit freier Pulspaaranordnung die Aufteilung von insgesamt sieben f2-Pulspaaren auf vier Panel und vier Blöcke mit variabler Anzahl von Slots unter der vereinfachten Annahme von einheitlichen Messzeiten für ausgewählte f2-Pulspaare gezeigt wurde, zeigt Fig. 8 eine Abwandlung, bei der keine einheitlichen Messzeiten vorausgesetzt werden.

Sobald ein Slot frei wird, weil für die bisher dort vermessene f2 der Mittelwert des Ldp mit hinreichendem SNR bestimmt wurde, wird er mit einer neuen f2 besetzt, wodurch sich ein neues Panel ergibt. Die Messzeit für einzelnen Panel kann sich dadurch verkürzen. Dies trifft insbesondere bei hörgeschädigten Patienten zu, weil für das neue Verfahren keine a priori Annahmen über das zu erwartende SNR bzw. die notwendige Messzeit der jeweiligen DPOAE benötigt werden.

## Patentansprüche

1. Verfahren zur Untersuchung des Hörvermögens für zumindest ein Ohr eines Säugetiers, in dem basierend auf der Messung von durch Paare von Anregungssignalen (f1, f2) evozierten DPOAE für unterschiedliche Anregungsfrequenzen f2 Wachstumskurven ermittelt werden, mit den Schritten:
dem Ohr werden erste Anregungssignale mit einer ersten Anregungsfrequenz f1 und einem ersten Schallpegel L1 und zweite Anregungssignale mit einer zweiten Anregungsfrequenz f2 und einem zweiten Schallpegel L2 gleichzeitig oder geringfügig zeitversetzt präsentiert; und
in dem Ohr werden Pulspaare mit einem ersten Puls des ersten Anregungssignals und einem zweiten Puls des zweiten Anregungssignals präsentiert und die dadurch evozierten DPOAE erfasst und ausgewertet;
**dadurch gekennzeichnet, dass**
ein Block, der zumindest zwei unterschiedliche Pulspaare mit unterschiedlichen zweiten Anregungsfrequenzen f2 aufweist, während einer Messdauer mehrfach wiederholt wird, wobei die Dauer des ersten und des zweiten Pulses in einem Pulspaar 2 bis 20 ms beträgt und wobei jeder Block von Pulspaaren während einer Blockzeit T_{B} präsentiert wird, die so gewählt ist, dass zwischen dem Beginn eines ersten und eines folgenden Pulspaares mit derselben Anregungsfrequenz f2 ein zeitlicher Abstand von 30 bis 100 ms liegt, wobei in einem Block der Beginn eines Pulspaares mit einem zeitlichen Abstand T auf den Beginn des im Block unmittelbar vorhergehenden Pulspaares folgt, wobei T mindestens der Länge des vorausgegangenen Pulses entspricht.

2. Verfahren nach einem der Anspruch 1, **dadurch gekennzeichnet, dass** in einem Block die zweiten Anregungsfrequenzen f2 von zwei unmittelbar aufeinander folgenden Pulspaaren mindestens eine Oktave auseinander liegen.

3. Verfahren nach einem der Ansprüche 1 bis 2, **dadurch gekennzeichnet, dass** während der Messdauer die gemessenen Schallpegel der DPOAE für Pulspaare gleicher zweiter Anregungsfrequenzen f2 gemittelt werden.

4. Verfahren nach einem der Ansprüche 1 bis 3, **dadurch gekennzeichnet, dass** der oder jeder Block von Pulspaaren während einer Blockzeit präsentiert wird, die so gewählt ist, dass zwischen dem Beginn eines ersten und eines folgenden Pulspaares mit derselben Anregungsfrequenz f2 ein zeitlicher Abstand von zumindest 70 ms liegt.

5. Verfahren nach einem der Ansprüche 1 bis 4, **dadurch gekennzeichnet, dass** zu Beginn der Messungen überprüft wird, ob die Frequenz fdp einer der DPOAE mit einer spontanen Emission (SOAE) interferiert.

6. Verfahren nach einem der Ansprüche 1 bis 5, **dadurch gekennzeichnet, dass** innerhalb eines Blocks die zweiten Schallpegel L2 der Pulspaare zueinander eine Pegeldifferenz aufweisen, die kleiner als 15 dB ist.

7. Verfahren nach einem der Ansprüche 1 bis 6, **dadurch gekennzeichnet, dass** die Abfolge der Pulspaare und der zeitliche Abstand zwischen zwei unmittelbar aufeinander folgenden Pulspaaren in einem Block konstant sind.

8. Verfahren nach einem der Ansprüche 1 bis 6, **dadurch gekennzeichnet, dass** bei Erreichen eines gewünschten Signal-Rausch-Verhältnisses für eine Anregungsfrequenz f2 die noch vorgesehenen Mittelungen für diese Anregungsfrequenz f2 übersprungen werden.

9. Verfahren nach einem der Ansprüche 1 bis 8, **dadurch gekennzeichnet, dass** die Schallpegel der DPOAE für alle in dem Block enthaltenen zweiten Anregungsfrequenzen f2 bei einem jeweils der Anregungsfrequenz f2 zugeordneten zweiten Schallpegel L2 gemessen und gemittelt werden, und die Messungen zumindest einmal für neue Schallpegel L2 durchgeführt werden.

10. Verfahren nach Anspruch 9, **dadurch gekennzeichnet, dass** für jede Anregungsfrequenz f2 eine Wachstumskurve aus Messwerten der Schallpegel der DPOAE für unterschiedliche Schallpegel L2 bestimmt wird, aus der dann die jeweiligen Schwellwerte bestimmt werden.

11. Verfahren nach Anspruch 10, **dadurch gekennzeichnet, dass** die Wachstumskurve aus zumindest drei bei drei unterschiedlichen Schallpegeln L2 aber derselben Anregungsfrequenz f2 bestimmten Werten für Schallpegel Ldp der DPOAE bestimmt wird, wobei die zumindest drei Schallpegel L2 einen oberen Schallpegel L2 umfassen, mit dessen Hilfe ein unterer Schallpegel L2 bestimmt wird, wobei der dritte, mittlere Schallpegel mit Hilfe des oberen und des unteren Schallpegels festgelegt wird.

12. Vorrichtung, eingerichtet zur Durchführung des Verfahrens nach einem der Ansprüche 1 bis 11,
die zumindest eine im/am Ohr zu platzierende Ohrsonde (11) mit einem oder zwei Miniaturlautsprechern (16) und einem Empfänger (17) umfasst, wobei der oder jeder Miniaturlautsprecher (16) zur Präsentation eines ersten Anregungssignales mit einer ersten Anregungsfrequenz f1 und einem ersten Schallpegel L1 und/oder eines zweiten Anregungssignales mit einer zweiten Anregungsfrequenz f2 und einem zweiten Schallpegel L2 ausgelegt ist, und wobei der Empfänger (17) zur Erfassung und Weiterleitung eines durch das erste und zweite Anregungssignal evozierten DPOAE ausgelegt ist, wobei ferner eine Rechnereinheit (15) vorgesehen ist, die dazu programmiert und eingerichtet ist, einen Satz von zumindest zwei Pulspaaren mit unterschiedlichen zweiten Anregungsfrequenzen f2 in einem Block zu präsentieren, der für eine Messdauer mehrfach wiederholt durchlaufen wird.

13. Hörgerät mit einer Vorrichtung nach Anspruch 12.

14. Verwendung des Verfahrens nach einem der Ansprüche 1 bis 11 und/oder der Vorrichtung nach Anspruch 12 in einem Verfahren nach einem der Ansprüche 1 bis 11 zur Einstellung eines Hörgerätes.

## Claims

1. Method for the examination of the hearing ability for at least one ear of a mammal, in which growth curves are determined on the basis of the measurement of DPOAE's evoked by pairs of excitation signals (f1, f2) for various excitation frequencies f2, including the steps:
the ear is presented with first excitation signals with a first excitation frequency f1 and a first noise level L1 and second excitation signals with a second excitation frequency f2 and a second noise level L2 at the same time or at temporal offset; and
pulse pairs with a first pulse of the first excitation signal and a second pulse of the second excitation signal are presented in the ear, and the DPOAE's evoked thereby are captured and evaluated;
**characterized in that**
a block which has at least two different pulse pairs with different second excitation frequencies f2, is repeated several times during a measuring period, wherein the duration of the first and the second pulse in a pulse pair is 2 to 20 ms, and wherein each block of pulse pairs is presented during a block time T_{B}, which is selected such that there is a time interval of 30 to 100 ms between the start of a first pulse pair and a subsequent pulse pair with the same excitation frequency f2, wherein, within a block, the start of a pulse pair follows the start of the immediately preceding pulse pair in the block by a time interval T, where T corresponds at least to the duration of the preceding pulse.

2. Method according to claim 1, **characterized in that**, in one block, the second excitation frequencies f2 of two immediately consecutive pulse pairs are at least one octave apart.

3. Method according to any one of claims 1 to 2, **characterized in that** the measured noise levels of the DPOAE's for pulse pairs of the same second excitation frequencies f2 are averaged during the measuring period.

4. Method according to any one of claims 1 to 3, **characterized in that** the or every block of pulse pairs during a block time is presented that is chosen in order to have a time interval of at least 70 ms, between the start of a first and a consecutive pulse pair with the same excitation frequency f2.

5. Method according to any one of claims 1 to 4, **characterized in that**, at the beginning of the measurements, a check occurs to determine whether the frequency fdp of one of the DPOAE's interferes with a spontaneous emission (SOAE).

6. Method according to any one of claims 1 to 5, **characterized in that**, within one block, the second noise levels L2 of the pulse pairs have a level difference from one another that is smaller than 15 dB.

7. Method according to any one of claims 1 to 6, **characterized in that** the sequence of the pulse pairs and the time interval between two immediately consecutive pulse pairs in a block remains constant.

8. Method according to any one of claims 1 to 6, **characterized in that**, once a desired signal-to-noise ratio for an excitation frequency f2 has been reached, the further averagings planned for this excitation frequency f2 are skipped.

9. Method according to any one of claims 1 to 8, **characterized in that** the noise levels of the DPOAE's for all the second excitation frequencies f2 contained in the block are measured and averaged for a second noise level L2 respectively allocated to the excitation frequency f2, and the measurements are conducted at least once for new noise levels L2.

10. Method according to claim 9, **characterized in that**, for each excitation frequency f2, a growth curve of measured values of the noise levels of the DPOAE's is determined for various noise levels L2, and the respective threshold values are then determined from said growth curves.

11. Method according to claim 10, **characterized in that** the growth curve is determined from at least three values for the noise level Ldp of the DPOAE determined at three different noise levels L2, but the same excitation frequency f2, wherein the at least three noise levels L2 include an upper noise level L2 that is used to determine a lower noise level L2, wherein the third, middle noise level is determined by means of the upper and the lower noise levels.

12. Device configured for executing the method according to any one of claims 1 to 11, comprising at least one ear probe (11) to be placed on/in the ear with one or two miniature loudspeakers (16) and one receiver (17), wherein the one or every miniature loudspeaker (16) is designed for the presentation of a first excitation signal with a first excitation frequency f1 and a first noise level L1 and/or a second excitation signal with a second excitation frequency f2 and a second noise level L2, and wherein the receiver (17) is designed for the capture and forwarding of a DPOAE evoked by the first and second excitation signal, wherein, furthermore, a computer unit (15) is provided that is programmed and configured to present a set of at least two pulse pairs with different second excitation frequencies f2 in a block, which is run repeatedly several times for one measuring period.

13. Hearing aid with a device according to claim 12.

14. Use of the method according to any one of claims 1 to 11 and/or the device according to claim 12 in a method according to any one of claims 1 to 11 for adjusting a hearing aid.

## Revendications

1. Procédé d'examen de la capacité auditive d'au moins une oreille d'un mammifère, dans lequel des courbes de croissance sont déterminées en se basant sur la mesure de DPOAE provoquées par des paires de signaux d'excitation (f1, f2) pour différentes fréquences d'excitation f2, comprenant les étapes ci-dessous :
des premiers signaux d'excitation avec une première fréquence d'excitation f1 et un premier niveau sonore L1 et des deuxièmes signaux d'excitation avec une deuxième fréquence d'excitation f2 et un deuxième niveau sonore L2 sont présentés à l'oreille simultanément ou de manière légèrement décalée dans le temps ; et
au sein de l'oreille, des paires d'impulsions sont présentées avec une première impulsion du premier signal d'excitation et une deuxième impulsion du deuxième signal d'excitation et les DPOAE ainsi provoquées sont enregistrées et évaluées ;
**caractérisé en ce que**
un bloc présentant au moins deux paires d'impulsions différentes avec des deuxièmes fréquences d'excitation f2 différentes est répété plusieurs fois pendant une période de mesure, dans lequel la durée des première et
deuxième impulsions dans une paire d'impulsions est comprise entre 2 et 20 ms et chaque bloc de paires d'impulsions est présenté pendant un temps de bloc T_{B} qui est choisi de sorte qu'un intervalle de temps compris entre 30 et 100 ms est présent entre le début d'une première paire d'impulsions et le début d'une paire d'impulsions suivante ayant la même fréquence d'excitation f2, dans lequel, au sein d'un bloc, le début d'une paire d'impulsions suit le début de la paire d'impulsions immédiatement précédente dans le bloc avec un intervalle de temps T, dans lequel T correspond au moins à la longueur de l'impulsion précédente.

2. Procédé selon la revendication 1, **caractérisé en ce que**, au sein d'un bloc, les deuxièmes fréquences d'excitation f2 de deux paires d'impulsions immédiatement consécutives sont espacées d'au moins une octave.

3. Procédé selon l'une quelconque des revendications 1 à 2, **caractérisé en ce que**, pendant la durée de mesure, les niveaux sonores mesurés des DPOAE sont moyennés pour des paires d'impulsions ayant la même deuxième fréquence d'excitation f2.

4. Procédé selon l'une quelconque des revendications 1 à 3, **caractérisé en ce que** le ou chaque bloc de paires d'impulsions est présenté pendant un temps de bloc qui est choisi de sorte qu'il y ait un intervalle de temps d'au moins 70 ms entre le début d'une première paire d'impulsions et le début d'une paire suivante ayant la même fréquence d'excitation f2.

5. Procédé selon l'une quelconque des revendications 1 à 4, **caractérisé en ce qu'**il est vérifié au début des mesures si la fréquence fdp d'une des DPOAE interfère avec une émission spontanée (SOAE).

6. Procédé selon l'une quelconque des revendications 1 à 5, **caractérisé en ce que**, au sein d'un bloc, les deuxièmes niveaux sonores L2 des paires d'impulsions présentent entre eux une différence de niveau inférieure à 15 dB.

7. Procédé selon l'une quelconque des revendications 1 à 6, **caractérisé en ce que** la séquence des paires d'impulsions et l'intervalle de temps entre deux paires d'impulsions immédiatement consécutives au sein d'un bloc sont constants.

8. Procédé selon l'une quelconque des revendications 1 à 6, **caractérisé en ce que**, lorsqu'un rapport signal/bruit souhaité est atteint pour une fréquence d'excitation f2, les moyennes prévues pour ladite fréquence d'excitation f2 sont ignorées.

9. Procédé selon l'une quelconque des revendications 1 à 8, **caractérisé en ce que** les niveaux sonores des DPOAE sont mesurés et moyennés pour toutes les deuxièmes fréquences d'excitation f2 contenues dans le bloc dans le cas d'un deuxième niveau sonore L2 associé respectivement à la fréquence d'excitation f2, et les mesures sont effectuées au moins une fois pour de nouveaux niveaux sonores L2.

10. Procédé selon la revendication 9, **caractérisé en ce que**, pour chaque fréquence d'excitation f2, une courbe de croissance est déterminée à partir de valeurs de mesure du niveau sonore des DPOAE pour différents niveaux sonores L2, et les valeurs de seuil respectives sont ensuite déterminées à partir de ladite courbe de croissance.

11. Procédé selon la revendication 10, **caractérisé en ce que** la courbe de croissance est déterminée à partir d'au moins trois valeurs de niveau sonore Ldp des DPOAE déterminées à trois niveaux sonores L2 différents mais à la même fréquence d'excitation f2, dans lequel les au moins trois niveaux sonores L2 comprennent un niveau sonore L2 supérieur à l'aide duquel un niveau sonore L2 inférieur est déterminé, dans lequel le troisième niveau sonore moyen est déterminé à l'aide des niveaux sonores supérieur et inférieur.

12. Dispositif conçu pour mettre en œuvre le procédé selon l'une quelconque des revendications 1 à 11, comprenant au moins une sonde auriculaire (11), à placer dans/sur l'oreille et munie de un ou deux haut-parleurs miniatures (16), et un récepteur (17), dans lequel le ou chaque haut-parleur miniature (16) est conçu pour présenter un premier signal d'excitation ayant une première fréquence d'excitation f1 et un premier niveau sonore L1 et/ou un deuxième signal d'excitation ayant une deuxième fréquence d'excitation f2 et un deuxième niveau sonore L2, et dans lequel le récepteur (17) est conçu pour enregistrer et transmettre une DPOAE provoqué par les premier et deuxième signaux d'excitation, dans lequel une unité de calcul (15) est en outre fournie, laquelle est programmée et conçue pour présenter un ensemble d'au moins deux paires d'impulsions ayant des deuxièmes fréquences d'excitation f2 différentes au sein d'un bloc, lequel ensemble est répété plusieurs fois pendant une période de mesure.

13. Appareil auditif comprenant un dispositif selon la revendication 12.

14. Utilisation du procédé selon l'une quelconque des revendications 1 à 11 et/ou du dispositif selon la revendication 12 dans un procédé selon l'une quelconque des revendications 1 à 11 permettant le réglage d'un appareil auditif.
